# EUROPEAN PATENT SPECIFICATION

(11) **EP 4 396 189 B1**
(45) Date of publication and mention of the grant of the patent: **13.08.2025**
(21) Application number: 22773182.5
(22) Date of filing: 02.09.2022
(51) Int. Cl.: C07D 493/12, C07D 493/18, C07D 519/00, A61K 31/357, A61P 35/00

(54) **IRON-ACTIVABLE NITROGEN-CONTAINING DERIVATIVES OF SALINOMYCIN AND NARASIN, SYNTHESIS AND USE FOR THE TREATMENT OF CANCERS**
SALINOMYCIN- UND NASARINDERIVATE MIT EINEM EISEN-AKTIVIERBAREN STICKSTOFF, DEREN SYNTHESE UND VERWENDUNG ZUR KREBSBEHANDLUNG
DÉRIVÉS DE SALINOMYCINE ET NASARINE AVEC UN AZOTE ACTIVABLE PAR DU FER, LEUR SYNTHÈSE ET UTILSATION POUR LE TRAITEMENT DU CANCER.

(30) Priority: 03.09.2021 EP 21306208
(43) Date of publication of application: 10.07.2024
(73) Proprietor: Centre National de la Recherche Scientifique, 75016 Paris (FR); Institut Curie, 75005 Paris (FR); Institut national de la santé et de la recherche médicale (INSERM), 75013 Paris (FR)
(72) Inventor: RODRIGUEZ, Raphaël, 30210 Vers-Pont-du-Gard (FR); CAÑEQUE, Tatiana, 75004 Paris (FR); MÜLLER, Sebastian, 94200 Ivry sur Seine (FR); ANTOSZCZAK, Michal, 62-003 Biedrusko (PL)
(74) Representative: Novagraaf Technologies
(86) International application number: PCT/EP2022/074451
(87) International publication number: WO 2023/031399

(56) References cited:
- EP-A1- 3 617 218
- WO-A1-2016/038223
- ANTOSZCZAK MICHAL ET AL: "Salinomycin and its derivatives - A new class of multiple-targeted "magic bullets"", EUROPEAN JOURNAL OF MEDICINAL CHEMISTRY, vol. 176, 9 May 2019 (2019-05-09), pages 208 - 227, XP085707340, ISSN: 0223-5234, DOI: 10.1016/J.EJMECH.2019.05.031
- DINIC JELENA ET AL: "Repurposing old drugs to fight multidrug resistant cancers", DRUG RESISTANCE UPDATES, CHURCHILL LIVINGSTONE, EDINBURGH, GB, vol. 52, 25 June 2020 (2020-06-25), XP086277117, ISSN: 1368-7646, [retrieved on 20200625], DOI: 10.1016/J.DRUP.2020.100713

## Description

The present invention concerns novel specific iron-activable nitrogen-containing derivatives of salinomycin and narasin, their synthesis and their uses in therapy notably for the treatment of cancer.

Resistance to chemotherapy and radiotherapy remains a major obstacle in the successful treatment of cancer. Resistance may occur during cancer treatment because of many reasons, such as some of the cancer cells which are not killed can mutate and become resistant, gene amplification resulting in the overexpression of a protein that renders the treatment ineffective may occur, or cancer cells may develop a mechanism to inactivate the treatment.

Cancer stem cells (CSCs) have been shown to be refractory to conventional therapeutic agents, can promote metastasis, and have been linked to cancer relapse. Salinomycin can selectively kill CSCs. Salinomycin derivatives accumulate in lysosomes and sequester iron in this organelle. As a result, accumulation of iron leads to the production of reactive oxygen species (ROS) and lysosomal membrane permeabilization, which in turn promotes cell death by ferroptosis (a recently characterized cell death mechanism i.e., reliant on iron and mediated by ROS). Thus, iron homeostasis in CSCs is key. This mechanism creates opportunities toward the development of next-generation therapeutics.

Salinomycin and ironomycin are potent antiproliferative drugs in the treatment of drug-resistant cancer cell lines (WO2016038223 and European Journal of Medicinal Chemistry 2019, vol.176, pages 208-227). However, these molecules present a carboxyl radical which is responsible for their degradation in the cells.

Therefore, there is a need for the development of novel and efficient anti-cancer drugs, which would be efficient while being specific for tumoral tissues. Especially, there is a need for novel and efficient anti-cancer drugs able to kill both cancer stem cells and therapy-resistant cancer cells.

The present invention proposes new molecules which aim to solve these needs:
Indeed, the inventors have prepared conjugates of salinomycin, narasin, ironomycin and other derivatives with dihydroartemisinin (DHA). Such conjugates are very interesting prodrugs: without being bound by any theory, they are able to penetrate the cells and the lysosomes, and in the lysosomes the active fraction is liberated whereas DHA is degraded. Thus, the therapeutic action can be exerted.

Consequently, the present invention first relates to a compound chosen from compounds of formula (I) and their diastereoisomers: 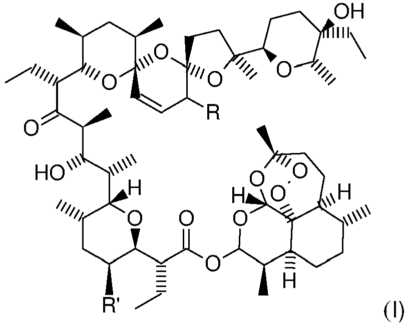
wherein R is a group -NR1R2 where R1 and R2 are identical or different and independently chosen from H, a C1-C20 alkyl group, a C3-C6 cycloalkyl group, an alkyne group and an aralkyl group, and
wherein R' is H or methyl.

Preferably R1 and R2 are identical or different, with the proviso that R1 and R2 are not simultaneously H.

Preferably R1 and R2 are different and independently chosen from H, a C1-C20 alkyl group, a C3-C6 cycloalkyl group, an alkyne group and an aralkyl group.

Preferably the compound is chosen from compounds of formula (I') and their diastereoisomers: 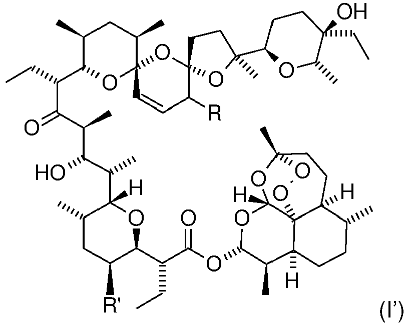 wherein R and R' are as defined above.

Preferably R1 and R2 are identical or different, with the proviso that R1 and R2 are not simultaneously H.

Preferably R1 and R2 are different and independently chosen from H, a C1-C20 alkyl group, a C3-C6 cycloalkyl group, an alkyne group and an aralkyl group.

When R' is H, then the compounds of formula (I) or (I') are conjugates of salinomycin. When R' is methyl, then the compounds of formula (I) or (I') are conjugates of narasin.

By diastereoisomers, it is meant epimers.

Indeed, R may be linked to the molecule by the up or down bond. In formula (I), the bond -R corresponds to one of the following bonds:

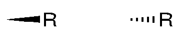

In other words, the diastereoisomers of formula (I) may be chosen from compounds of formula (II) and (III): 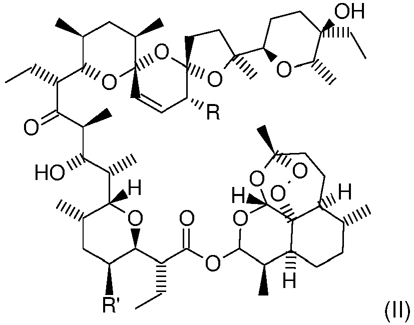 and

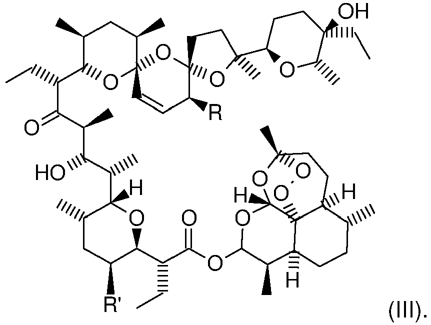

The diastereoisomers of formula (I') may be chosen from compounds of formula (II') and (III'): 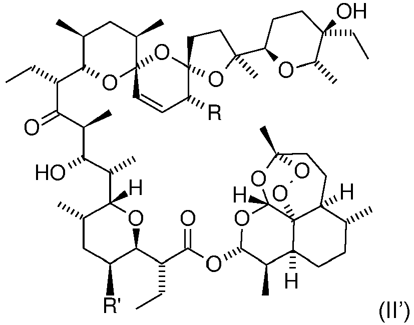 and

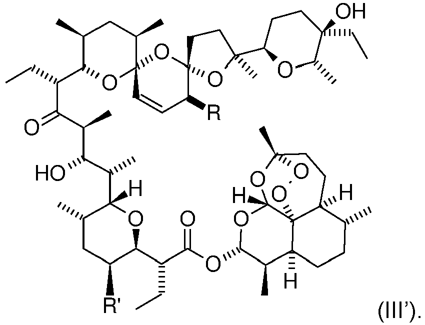

By "C1-C20 alkyl", it is meant a linear or hydrocarbon group comprising from 1 to 20 carbon atoms, preferably 2 to 15 carbon atoms, or a branched hydrocarbon group comprising from 3 to 20 carbon atoms, preferably 3 to 10 carbon atoms. Examples of C1-C20 alkyl groups include methyl, ethyl, n-propyl, isopropyl, n-butyl, isobutyl, tert-butyl, n-pentyl, isopentyl, n-hexyl, octyl or dodecyl groups, and preferably ethyl, n-propyl, n-butyl, isobutyl, n-pentyl, n-hexyl or dodecyl groups.

By "C3-C6 cycloalkyl", it is meant a cyclic hydrocarbon group comprising from 3 to 6 carbon atoms. Examples of C3-C6 cycloalkyl groups include cyclopropyl, cyclobutyl, cyclopentyl, and cyclohexyl groups. More preferably, the C3-C6 cycloalkyl is cyclopropyl.

By "alkyne group", it is meant an unsaturated hydrocarbon comprising at least one carbon-carbon triple bond (-C≡C-). Preferably the alkyne group is linear. Preferably the alkyne group comprises 2 to 10 carbon atoms, preferably 2 to 6 carbon atoms. Preferably the alkyne group comprises 3 carbon atoms. Preferably the alkyne group is propynyl.

By "aralkyl", it is meant a group -(CH₂)n-aryl, wherein n is an integer from 1 to 5, and aryl is a monocyclic or polycyclic aromatic hydrocarbon group, which may be optionally substituted. Preferably, the aryl is a phenyl. The aryl may be substituted by at least one group chosen from a hydroxyl group, a C1-C20 alkyl group (preferably a C1-C6 alkyl group) and a halogen. By "halogen", it is meant fluorine (F), chlorine (CI), iodine (I) or bromine (Br). Preferably, the aryl is substituted, preferably in para, by at least one group chosen from a hydroxyl group, a C1-C6 alkyl group such as a methyl, and a halogen such as F or CI. Preferably, the aralkyl group is a benzyl which is not substituted, or substituted, preferably in para, by at least one group chosen from a hydroxyl group, a C1-C6 alkyl group such as a methyl, and a halogen such as F or CI.

Preferably, the compounds of the invention are of formula (II'): 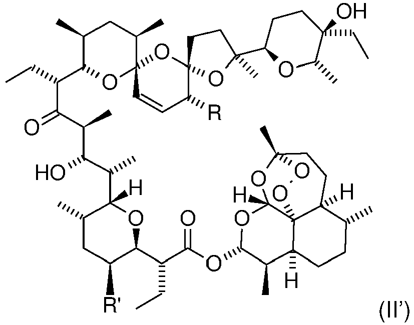 wherein R and R' are as defined above.

Preferably, alternatively, the compounds of the invention are of formula (III'): 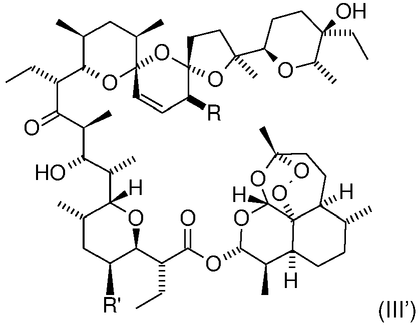 wherein R is as defined above and R' is H.

Preferably R is a hydroxyl group or a group -NR1R2, where R1 is chosen from a C1-C20 alkyl group, a C3-C6 cycloalkyl group, an alkyne group and an aralkyl group, and R2 is chosen from H, a C1-C20 alkyl group and an alkyne group.

Preferably R is a hydroxyl group or a group -NR1R2 where R1 and R2 are different and independently chosen from H, a C3-C6 cycloalkyl group and an alkyne group.

Preferably R is a hydroxyl group.

Alternatively, preferably R is -NR1R2. In this case, preferably R2 is H and R1 is a C3-C6 cycloalkyl group or an alkyne group, preferably a C2-C6 alkyne group. Preferably, R2 is H and R1 is cyclopropyl or propynyl.

Preferably, the compound of formula (I) of the invention is a compound of formula (I'). Preferably it is chosen from the following compounds: 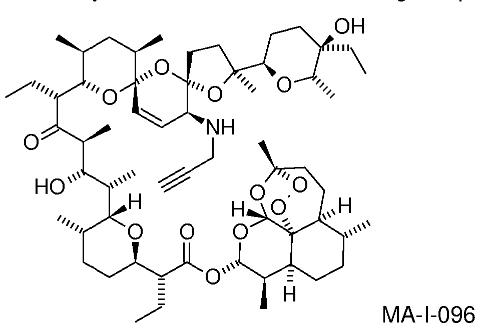 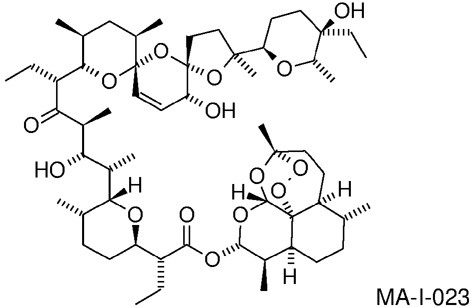 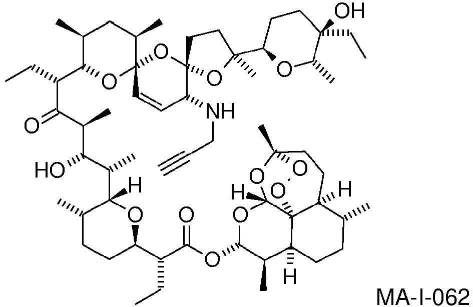 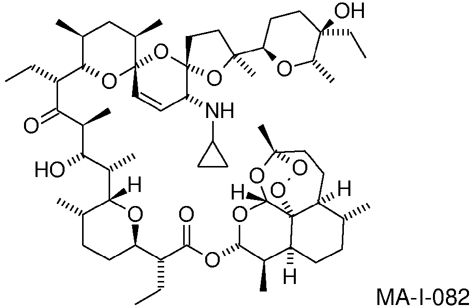 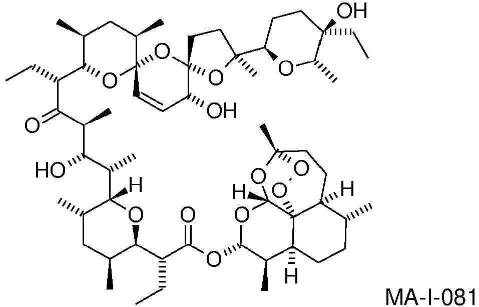 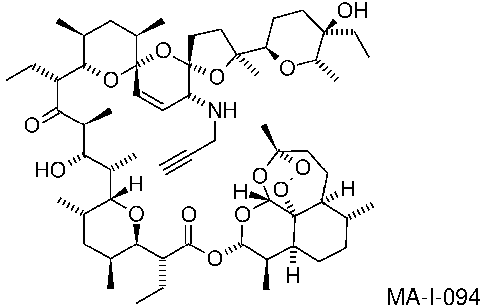 and

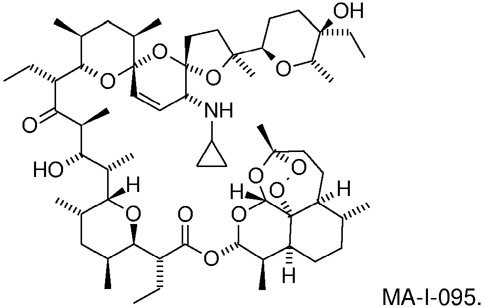

As shown in the examples, the compounds of formula (I) of the invention are very specific for cancerous cells of various cancers as compared to non-cancerous cell lines. Moreover, as shown in Table 1, the compounds of formula (I) of the invention show an anti-cancer activity which is selective.

### Preparation of the compounds of the invention

The compounds of the invention may be prepared particularly by the following process, which is illustrated in the examples:
Salinomycin or narasin, or one of their respective C20-amine derivatives, is mixed with dihydroartemisinin (DHA) in an anhydrous medium. Then 1-ethyl-3-(3-dimethylaminopropyl)carbodiimide hydrochloride (EDCI) and 4-dimethylaminopyridine (DMAP) are added, and the resulting mixture is stirred, in order to obtain the compound of formula (I).

Alternatively, the DHA-conjugated prodrugs of the invention may be prepared using two other protocols, which are described below:
1. DCC/DMAP/DIPEA-activated protocol:
   Salinomycin or narasin, or one of their respective C20-amine derivatives, in anhydrous CH₂Cl₂, preferably at low temperature (i.e. around 0°C), is mixed with *N*,*N'-*dicyclohexylcarbodiimide (DCC), DMAP and DHA. Then, *N*,*N*-diisopropylethylamine (DIPEA) is added. After incubation, the reaction is warmed to room temperature, and stirred for sufficient time, preferably a few hours, preferably around 24 h.
2. DCC/PPy/pTSA-activated protocol:
   To a solution of salinomycin or narasin, or one of their respective C20-amine derivatives in anhydrous CH₂Cl₂, the following reagents are added: DCC, 4-pyrrolidinopyridine (PPy), DHA and catalytic p-toluenesulfonic acid monohydrate (*p*TSA). The resulting mixture is then stirred. The resulting mixture is preferably first stirred at a low temperature (i.e. around 0°C) for a few hours, preferably around 6 h, and then at room temperature for a few hours, preferably around 18 h.

### Composition and uses

The present invention also relates to a composition comprising, in a pharmaceutically acceptable medium, at least one compound of formula (I) according to the invention.

The present invention also relates to the use of a compound of formula (I) according to the invention as a medicament.

The present invention also relates to the use of a compound of formula (I) according to the invention for preventing and/or treating cancer.

### Anti-cancer use

The compounds of formula (I) of the invention may be used for preventing and/or treating cancer.

By "preventing", it is meant avoiding the cancer to occur.

By "treatment", it is meant the curative treatment of cancer. A curative treatment is defined as a treatment that completely treat (cure) or partially treat cancer (i.e. induces tumor growth stabilization, retardation or regression).

The "subject" refers to any subject and typically designates a patient, preferably a subject undergoing a treatment of cancer such as immunotherapy, chemotherapy and/or radiotherapy. In any case, the subject is preferably a vertebrate, more preferably a mammal, even more preferably a human being.

By "cancer", it is meant any type of cancer. The cancer may be solid or non-solid, and may be for example selected from a colon cancer, a colorectal cancer such as colorectal cancers with a BRAF mutation (especially BRAF V600E), a melanoma, a bone cancer, a breast cancer such as triple-negative breast cancer (i.e. breast cancer that tests negative for estrogen receptors, progesterone receptors, and excess HER2 protein), a thyroid cancer, a prostate cancer, an ovarian cancer, a lung cancer, a pancreatic cancer, a glioma such as a glioblastoma, a cervical cancer, an endometrial cancer, a head and neck cancer, a liver cancer, a bladder cancer, a renal cancer, a skin cancer, a stomach cancer, a testis cancer, an urothelial cancer or an adrenocortical carcinoma, leukemia such as acute myeloid leukemia, but also non-solid cancers such as lymphoma or multiple myeloma.

Preferably, the cancer is a colon cancer, a colorectal cancer such as colorectal cancers with a BRAF mutation (especially BRAF V600E), a breast cancer such as triple-negative breast cancer (i.e. breast cancer that tests negative for estrogen receptors, progesterone receptors, and excess HER2 protein), a pancreatic cancer, a glioma such as a glioblastoma, leukemia such as acute myeloid leukemia, lymphoma or multiple myeloma.

The cancer can be a metastatic cancer or not. A typical cancer is a cancer resistant to the first-line chemotherapy.

The invention also relates to the use of at least one compound of formula (I) for increasing the sensitivity of a cancer to a chemotherapeutic drug.

A further object of the invention is the use of at least one compound of formula (I) for decreasing the resistance of a cancer with respect to a chemotherapeutic drug.

The invention also relates to a product comprising:
a) at least one compound of formula (I) of the invention, and
b) at least one additional therapy,
as a combination product for a simultaneous, separate or sequential use for treating cancer, and/or for preventing cancer metastasis, and/or for preventing cancer recurrence, and/or for decreasing resistance to the additional therapy b), in a subject.

It also relates to the use of at least one compound of formula (I) of the invention, for preventing and/or treating a cancer in combination or in association with at least one additional therapy.

It further relates to the use of at least one compound of formula (I) of the invention, for preventing and/or treating a cancer in a subject treated by at least one additional therapy. The invention also relates to at least one compound of formula (I) of the invention, for use as an adjuvant cancer therapy. An adjuvant therapy is a therapy for treating cancer that is given besides a primary or initial therapy ("first-line therapy"), to maximize its effectiveness.

Said additional therapy b) may be immunotherapy, chemotherapy and/or radiotherapy. Preferably the additional therapy b) is immunotherapy and/or chemotherapy.

By "immunotherapy", it is meant a therapy with is able to induce, enhance or suppress an immune response. Said immunotherapy is preferably chosen from cytokines, chemokines, growth factors, growth inhibitory factors, hormones, soluble receptors, decoy receptors; monoclonal or polyclonal antibodies, mono-specific, bi-specific or multi-specific antibodies, monobodies, polybodies; vaccination; or adoptive specific immunotherapy.

Preferably the immunotherapy is chosen from monoclonal or polyclonal antibodies, mono-specific, bi-specific or multi-specific antibodies, monobodies, polybodies, such as anti-angiogenic agents like Bevacuzimab (mAb, inhibiting VEGF-A, Genentech); IMC-1121B (mAb, inhibiting VEGFR-2, ImClone Systems); CDP-791 (Pegylated DiFab, VEGFR-2, Celltech); 2C3 (mAb, VEGF-A, Peregrine Pharmaceuticals); VEGF-trap (soluble hybrid receptor VEGF-A, PIGF (placenta growth factor) Aventis/Regeneron).

Preferably the immunotherapy is a monoclonal antibody, preferably an anti-checkpoint antibody.

The anti-checkpoint antibodies comprise antibodies directed against an immune checkpoint, which may be chosen from PD1, PDL1, PDL2, CTLA4, BTLA, CD27, CD40, OX40, GITR (also called "Tumor necrosis factor receptor superfamily member 18" or TNFRSF18), CD137 (also called 4-1BB or TNFRS9), CD28, ICOS, IDO (indoleamine 2,3-dioxygenase), B7H3 (also called CD276), KIR2DL2 (also called killer cell immunoglobulin-like receptor 2DL2), NKG2 (a family of the C-type lectin receptors), LAG3 (also called Lymphocyte Activation Gene-3) and CD70. Preferably the anti-checkpoint antibodies are anti-PD1, anti-PDL1, anti-PDL2 or anti-CTLA4 antibodies. Anti-PD1 antibodies include nivolumab and pembrolizumab. Anti-CTLA4 antibodies include ipilimumab and tremelimumab.

The "chemotherapy" or "chemotherapeutic agent" refers to compounds which are used in the treatment of cancer and that have the functional property of inhibiting a development or progression of a neoplasm in a human, particularly a malignant (cancerous) lesion. Chemotherapeutic agents have different modes of actions, for example, by influencing either DNA or RNA and interfering with cell cycle replication.

Examples of chemotherapeutic agents that act at the DNA level or on the RNA level are:
- anti-metabolites, such as Azathioprine, Cytarabine, Fludarabine phosphate, Fludarabine, Gemcitabine, cytarabine, Cladribine, capecitabine 6-mercaptopurine, 6-thioguanine, methotrexate, 5-fluorouracil and hydroxyurea;
- alkylating agents, such as Melphalan, Busulfan, Cisplatin, Carboplatin, Cyclophosphamide, Ifosphamide, Dacarabazine, Fotemustine, Procarbazine, Chlorambucil, Thiotepa, Lomustine, Temozolomide;
- anti-mitotic agents, such as Vinorelbine, Vincristine, Vinblastine, Docetaxel, Paclitaxel;
- topoisomerase inhibitors, such as Doxorubicin, Amsacrine, Irinotecan, Daunorubicin, Epirubicin, Mitomycin, Mitoxantrone, Idarubicin, Teniposide, Etoposide, Topotecan;
- antibiotics, such as actinomycin and bleomycin;
- asparaginase;
- anthracyclines or taxanes.

Other chemotherapeutic agents are tyrosine kinase inhibitors (TKIs). A number of TKls are in late and early stage development for treatment of various types of cancer. Exemplary TKIs include, but are not limited to: BAY 43-9006 (Sorafenib, Nexavar^{®}) and SU11248 (Sunitinib, Sutent^{®}), Imatinib mesylate (Gleevec^{®}, Novartis); Gefitinib (Iressa^{®}, AstraZeneca); Erlotinib hydrochloride (Tarceva^{®}, Genentech); Vandetanib (Zactima^{®}, AstraZeneca), Tipifarnib (Zarnestra^{®}, Janssen-Cilag); Dasatinib (Sprycel^{®}, Bristol Myers Squibb); Lonafarnib (Sarasar^{®}, Schering Plough); Vatalanib succinate (Novartis, Schering AG); Lapatinib (Tykerb^{®}, GlaxoSmithKline); Nilotinib (Novartis); Lestaurtinib (Cephalon); Pazopanib hydrochloride (GlaxoSmithKline); Axitinib (Pfizer); Canertinib dihydrochloride (Pfizer); Pelitinib (National Cancer Institute, Wyeth); Tandutinib (Millennium); Bosutinib (Wyeth); Semaxanib (Sugen, Taiho); AZD-2171 (AstraZeneca); VX-680 (Merck, Vertex); EXEL-0999 (Exelixis); ARRY-142886 (Array BioPharma, AstraZeneca); PD-0325901 (Pfizer); AMG-706 (Amgen); BIBF-1120 (Boehringer Ingelheim); SU-6668 (Taiho); CP-547632 (OSI); (AEE-788 (Novartis); BMS-582664 (Bristol-Myers Squibb); JNK-401 (Celgene); R-788 (Rigel); AZD-1152 HQPA (AstraZeneca); NM-3 (Genzyme Oncology); CP-868596 (Pfizer); BMS-599626 (Bristol-Myers Squibb); PTC-299 (PTC Therapeutics); ABT-869 (Abbott); EXEL-2880 (Exelixis); AG-024322 (Pfizer); XL-820 (Exelixis); OSI-930 (OSI); XL-184 (Exelixis); KRN-951 (Kirin Brewery); CP-724714 (OSI); E-7080 (Eisai); HKI-272 (Wyeth); CHIR-258 (Chiron); ZK-304709 (Schering AG); EXEL-7647 (Exelixis); BAY-57-9352 (Bayer); BIBW-2992 (Boehringer Ingelheim); AV-412 (AVEO); YN-968D1 (Advenchen Laboratories); Staurosporin, Midostaurin (PKC412, Novartis); Perifosine (AEterna Zentaris, Keryx, National Cancer Institute); AG-024322 (Pfizer); AZD-1152 (AstraZeneca); ON-01910Na (Onconova); and AZD-0530 (AstraZeneca).

Herein described are also (i) a method for preventing or treating cancer, (ii) a method for increasing the sensitivity of a cancer to a chemotherapeutic agent, and (iii) a method for decreasing the resistance of a cancer with respect to a chemotherapeutic drug, each of said methods comprising administering to a subject in need thereof with an effective amount of at least one compound of formula (I) as defined above, preferably together with a chemotherapeutic drug.

The compound of formula (I) of the invention is preferably administered at a therapeutically effective amount or dose. As used herein, "a therapeutically effective amount or dose" refers to an amount of the compound of the invention which prevents, removes, slows down the disease, or reduces or delays one or several symptoms or disorders caused by or associated with said disease in the subject, preferably a human being. The effective amount, and more generally the dosage regimen, of the compound of the invention and pharmaceutical compositions thereof may be determined and adapted by the one skilled in the art. An effective dose can be determined by the use of conventional techniques and by observing results obtained under analogous circumstances. The therapeutically effective dose of the compound of the invention will vary depending on the disease to be treated or prevented, its gravity, the route of administration, any co-therapy involved, the patient's age, weight, general medical condition, medical history, etc.

Typically, the amount of the compound to be administered to a patient may range from about 0.01 to 500 mg/kg of body weight for a human patient. In a particular embodiment, the pharmaceutical composition according to the invention comprises 0.01 mg/kg to 300 mg/kg of the compound of the invention, preferably from 0.01 mg/kg to 3 mg/kg, for instance from 25 to 300 mg/kg.

In a particular aspect, the compounds of the invention can be administered to the subject by parenteral route, topical route, oral route or intravenous injection. The compound or the nanoparticle of the invention may be administered to the subject daily (for example 1, 2, 3, 4, 5, 6 or 7 times a day) during several consecutive days, for example during 2 to 10 consecutive days, preferably from 3 to 6 consecutive days. Said treatment may be repeated during 1, 2, 3, 4, 5, 6 or 7 weeks, or every two or three weeks or every one, two or three months. Alternatively, several treatment cycles can be performed, optionally with a break period between two treatment cycles, for instance of 1, 2, 3, 4 or 5 weeks. The compound of the invention can for example be administered as a single dose once a week, once every two weeks, or once a month. The treatment may be repeated one or several times per year. Doses are administered at appropriate intervals which can be determined by the skilled person. The amount chosen will depend on multiple factors, including the route of administration, duration of administration, time of administration, the elimination rate of the compound, or of the various products used in combination with said compound, the age, weight and physical condition of the patient and his/her medical history, and any other information known in medicine.

The administration route can be oral, topical or parenteral, typically rectal, sublingual, intranasal, intra-peritoneal (IP), intra-venous (IV), intra-arterial (IA), intra-muscular (IM), intra-cerebellar, intrathecal, intratumoral and/or intradermal. The pharmaceutical composition is adapted for one or several of the above-mentioned routes. The pharmaceutical composition is preferably administered by injection or by intravenous infusion of suitable sterile solutions, or in the form of liquid or solid doses via the alimentary canal.

The present invention also relates to a composition comprising, in a pharmaceutically acceptable medium, at least one compound of formula (I) according to the invention. Such a composition comprises a pharmaceutically acceptable medium (or carrier).

The carrier must be "acceptable" in the sense of being compatible with the other ingredients of the formulations and not deleterious to the recipient thereof.

The pharmaceutical composition can be formulated as solutions in pharmaceutically compatible solvents or as gels, oils, emulsions, suspensions, or dispersions in suitable pharmaceutical solvents or vehicles, or as pills, tablets, capsules, powders, suppositories, etc. that contain solid vehicles in a way known in the art, possibly through dosage forms or devices providing sustained and/or delayed release. For this type of formulation, an agent such as cellulose, lipids, carbonates or starches are used advantageously.

Agents or vehicles that can be used in the formulations (liquid and/or injectable and/or solid) are excipients or inert vehicles, i.e. pharmaceutically inactive and non-toxic vehicles.

Mention may be made, for example, of saline, physiological, isotonic and/or buffered solutions, compatible with pharmaceutical use and known to those skilled in the art. The compositions may contain one or more agents or vehicles chosen from dispersants, solubilizers, stabilizers, preservatives, etc.

Particular examples are methylcellulose, hydroxymethylcellulose, carboxymethylcellulose, cyclodextrins, polysorbate 80, mannitol, gelatin, lactose, liposomes, vegetable oils or animal, acacia, etc. Preferably, vegetable oils are used.

Formulations of the present invention suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion.

Formulations suitable for parenteral administration conveniently comprise a sterile oily or aqueous preparation of the active ingredient which is preferably isotonic with the blood of the recipient. Every such formulation can also contain other pharmaceutically compatible and non-toxic auxiliary agents, such as, e.g. stabilizers, antioxidants, binders, dyes, emulsifiers or flavoring substances.

The invention is now illustrated by the following examples.

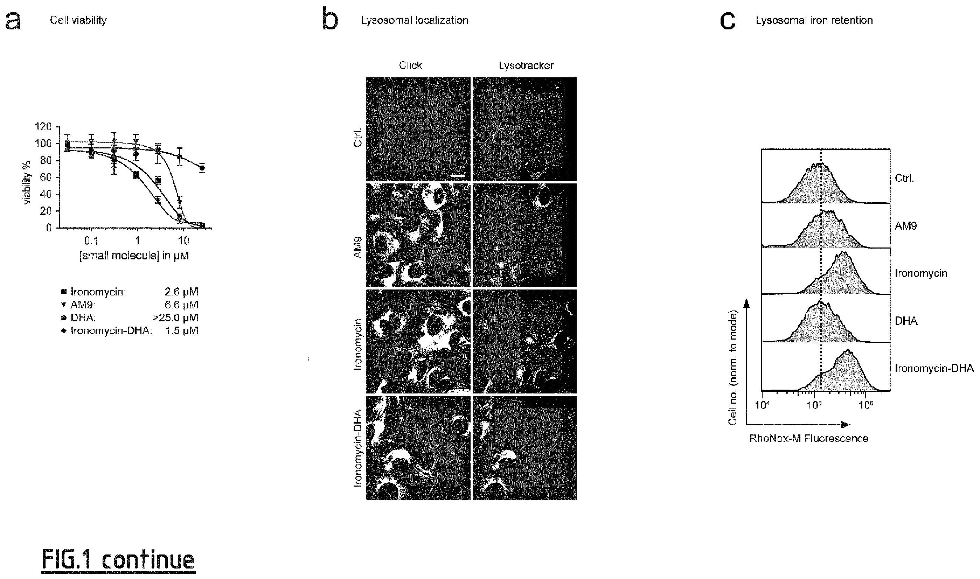

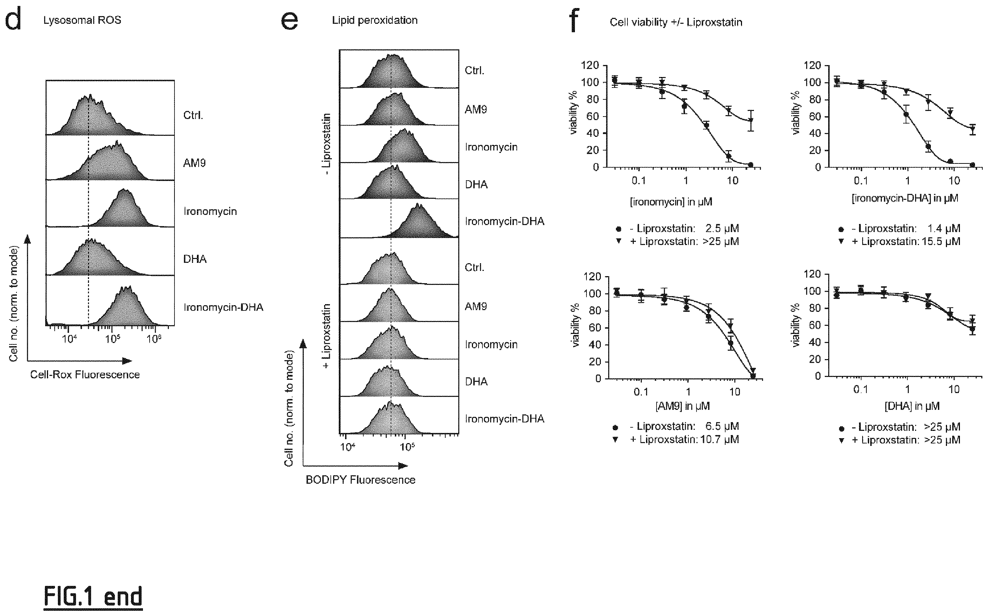

### The legend of Figure 1 is as follows:

### Figure 1

(a) Cell viability curves of cells treated with ironomycin, AM9, DHA or ironomycin-DHA prodrug of the invention (ironomycin-DHA).
(b) Fluorescence images of cells treated with ironomycin, AM9 or ironomycin-DHA prodrug of the invention (ironomycin-DHA) and lysotracker. Error bar = 10 µm.
(c) Flow cytometry histograms of RhoNox-M fluorescence of cells treated with ironomycin, AM9, DHA or ironomycin-DHA prodrug of the invention (ironomycin-DHA) (1 µM, 24 h).
(d) Flow cytometry histograms of CellRox fluorescence of cells treated with ironomycin, AM9, DHA or ironomycin-DHA prodrug of the invention (ironomycin-DHA) (1 µM, 24 h).
(e) Flow cytometry histograms of BODIPY-C11 green fluorescence of cells treated with ironomycin, AM9, DHA or ironomycin-DHA prodrug of the invention (ironomycin-DHA) (1 µM, 24 h). Cells were treated DHA in the presence or absence of liproxstatin for 24 h.
(f) Cell viability curves of cells treated with ironomycin, AM9, DHA or ironomycin-DHA prodrug of the invention (ironomycin-DHA) in the presence or absence of liproxstatin.

All data were obtained using the pancreatic ductal adenocarcinoma cell line PDAC053T.

### Examples

### 1/ General procedure for the synthesis of dihydroartemisinin-conjugated prodrugs (compounds of formula (I) according to the invention)

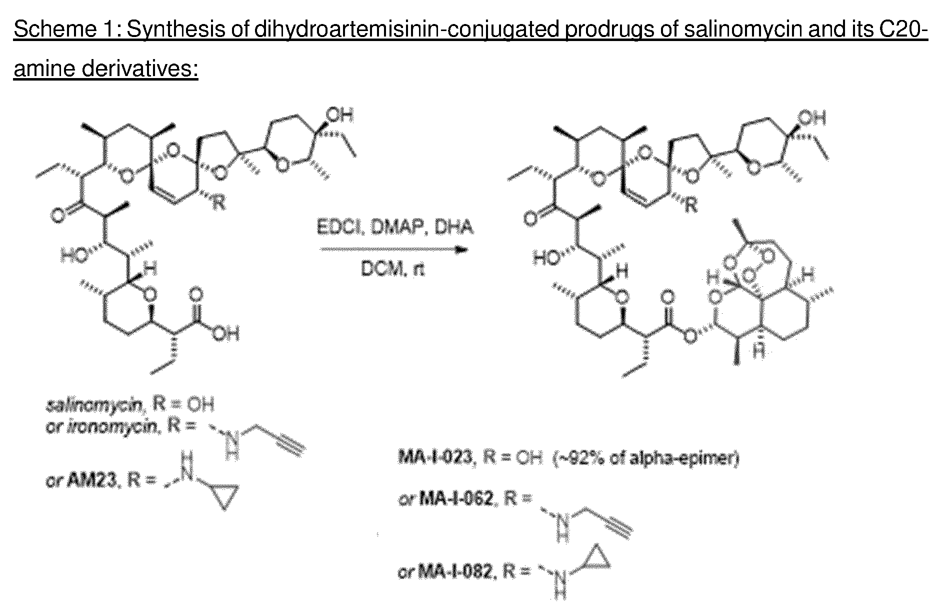

To a stirred solution of salinomycin (1.0 equiv.) or its respective C20-amine derivative (ironomycin **AM5** or **AM23,** 1.0 equiv.) and dihydroartemisinin (2.0 equiv.) in anhydrous CH₂Cl₂, was added EDCI hydrochloride (1.2 equiv.) and DMAP (0.5 equiv.) in one portion. The resulting mixture was stirred at room temperature for 48 h. After that, the reaction mixture was evaporated in vacuo and purified chromatographically on silica gel using the CombiFlash system and/or HPLC equipped with a C18-reverse phase column (gradient: ACN/H₂O/FA 4/1/0.1 to ACN/FA 1/0.1 for **MA-I-023,** or ACN/H₂O/FA 1/1/0.1 to ACN/FA 1/0.1 for **MA-I-062** and **MA-I-082)** to give the pure products of the reaction (17-31% yield) as white or cream amorphous solids.

**Salinomycin-dihydroartemisinin prodrug MA-I-023 (compound of formula (I) according to the invention)**: Yield: 153 mg, 31%. Isolated as a white amorphous solid, >95% pure by NMR, and two spots by TLC (~92% of α-epimer); R_{f}: 0.64 and 0.71 in cyclohexane/EtOAc 50%. Strain green with PMA; ¹H NMR (400 MHz, CD₂Cl₂) δ 6.40 (d, J = 9.8 Hz, 1H), 6.01 (dd, J = 10.8, 2.2 Hz, 1H), 5.91 (dd, J = 10.7, 1.2 Hz, 1H), 5.77 (s, 1H), 4.13 (dd, J = 11.2, 6.1 Hz, 1H), 4.02 (dd, J = 11.0, 1.8 Hz, 1H), 3.94 (d, J = 5.2 Hz, 1H), 3.86-3.68 (m, 3H), 3.61 (dd, J = 9.8, 1.4 Hz, 1H), 3.52 (dd, J = 10.5, 1.7 Hz, 1H), 3.20 (dq, J = 10.1, 7.2 Hz, 1H), 3.03 (td, J = 11.3, 3.7 Hz, 1H), 2.68 (ddd, J = 13.0, 7.6, 2.9 Hz, 2H), 2.49 (dqd, J = 9.9, 7.1, 4.4 Hz, 1H), 2.41-2.16 (m, 4H), 2.15-0.60 (m, 71H) ppm; ¹³C NMR (101 MHz, CD₂Cl₂) δ 214.4, 174.5, 133.3, 121.0, 106.7, 104.3, 99.4, 93.8, 92.3, 88.1, 81.4, 80.8, 77.5, 74.8, 74.5, 72.3, 71.1, 68.8, 67.8, 57.3, 52.0, 50.1, 48.4, 46.4, 40.7, 39.2, 37.0, 36.9, 36.7, 36.6, 34.8, 33.6, 33.1, 31.0, 30.6, 29.5, 28.4, 26.5, 26.1, 25.9, 24.9, 22.6, 22.5, 21.9, 20.8, 20.2, 19.6, 17.4, 15.7, 14.8, 14.2, 13.6, 12.7, 12.5, 10.9, 7.4, 6.5 ppm; HRMS (ESI⁺) m/z [M+NH₄]⁺ Calcd for C₅₇H₉₆NO₁₅ 1034.6780, Found 1034.6765.

**Ironomycin-dihydroartemisinin prodrug MA-I-062 (compound of formula (I) according to the invention)**: Yield: 17 mg, 30%. Isolated as a white amorphous solid, >95% pure by NMR, and a single spot by TLC; R_{f}: 0.59 in cyclohexane/EtOAc 50%. Strain green with PMA; ¹H NMR (500 MHz, CD₂Cl₂) δ 6.41 (d, J = 9.7 Hz, 1H), 6.03 (dd, J = 22.7, 10.8 Hz, 2H), 5.79 (s, 1H), 4.14 (dd, J = 11.2, 6.1 Hz, 1H), 4.04 (d, J = 10.3 Hz, 1H), 3.78 (q, J = 6.9 Hz, 1H), 3.61 (td, J = 10.0, 1.7 Hz, 2H), 3.57-3.44 (m, 3H), 3.24 (dq, J = 9.8, 7.3 Hz, 1H), 3.15 (s, 1H), 3.05 (td, J = 11.3, 3.7 Hz, 1H), 2.76-2.68 (m, 1H), 2.49 (tdd, J = 14.3, 7.1, 4.6 Hz, 2H), 2.38-2.26 (m, 3H), 2.23 (s, 1H), 2.20-0.60 (m, 73H) ppm; ¹³C NMR (126 MHz, CD₂Cl₂) δ 214.4, 174.7, 131.8, 120.7, 106.8, 104.3, 99.0, 93.9, 92.3, 87.7, 82.9, 81.9, 80.9, 77.4, 74.9, 74.1, 72.2, 71.1, 70.9, 68.7, 57.5, 55.9, 52.0, 50.1, 48.7, 46.4, 40.8, 39.3, 37.6, 37.0, 36.9, 36.8, 36.7, 34.8, 33.6, 32.9, 31.3, 30.9, 29.5, 28.4, 26.5, 25.9, 25.5, 24.9, 22.4, 21.9, 21.7, 20.2, 19.6, 17.3, 15.7, 14.6, 14.1, 13.6, 12.7, 12.5, 10.9, 7.3, 6.5 ppm, one signal overlapped; HRMS (ESI⁺) m/z [M+H]⁺ Calcd for C₆₀H₉₆NO₁₄ 1054.6831, Found 1054.6815.

**C20-cyclopropylaminosalinomycin-dihydroartemisinin prodrug MA-I-082 (compound of formula (I) according to the invention):** Yield: 14 mg, 26%. Isolated as a cream amorphous solid, >95% pure by NMR, and a single spot by TLC; R_{f}: 0.74 in cyclohexane/EtOAc 50%. Strain green with PMA; ¹H NMR (500 MHz, CD₂Cl₂) δ 6.41 (d, J = 9.6 Hz, 1H), 6.03 (s, 2H), 5.79 (s, 1H), 4.13 (dd, J = 10.5, 6.3 Hz, 1H), 4.03 (d, J = 10.0 Hz, 1H), 3.72 (dd, J = 12.8, 6.1 Hz, 1H), 3.61 (d, J = 9.7 Hz, 1H), 3.52 (d, J = 9.0 Hz, 1H), 3.46 (d, J = 10.2 Hz, 1H), 3.25 (dt, J = 16.1, 7.9 Hz, 1H), 3.17 (s, 1H), 3.04 (t, J = 10.4 Hz, 1H), 2.72 (d, J = 10.7 Hz, 1H), 2.54-2.46 (m, 2H), 2.35-2.26 (m, 3H), 2.20-0.60 (m, 74H), 0.41 (ddd, J = 18.2, 11.1, 6.2 Hz, 3H), 0.28 (d, J = 7.6 Hz, 1H) ppm; ¹³C NMR (126 MHz, CD₂Cl₂) δ 214.5, 174.7, 132.6, 120.7, 107.2, 104.3, 98.9, 93.9, 92.4, 87.4, 82.0, 80.9, 77.5, 74.9, 73.5, 72.3, 71.2, 68.7, 57.6, 56.6, 52.1, 50.2, 48.7, 46.4, 40.9, 39.4, 37.0, 36.9, 36.8, 36.3, 34.8, 33.7, 33.0, 31.9, 30.9, 29.6, 29.4, 28.5, 26.6, 25.9, 25.0, 24.9, 22.5, 22.2, 21.9, 21.8, 20.2, 19.7, 17.4, 15.8, 14.5, 14.1, 13.6, 12.7, 12.5, 10.9, 7.4, 7.3, 6.6, 6.5 ppm; HRMS (ESI⁺) m/z [M+H]⁺ Calcd for C₆₀H₉₈NO₁₄ 1056.6987, Found 1056.6997.

### 2/ Synthesis of dihydroartemisinin-conjugated prodrug MA-I-096 (compound of formula (I) according to the invention)

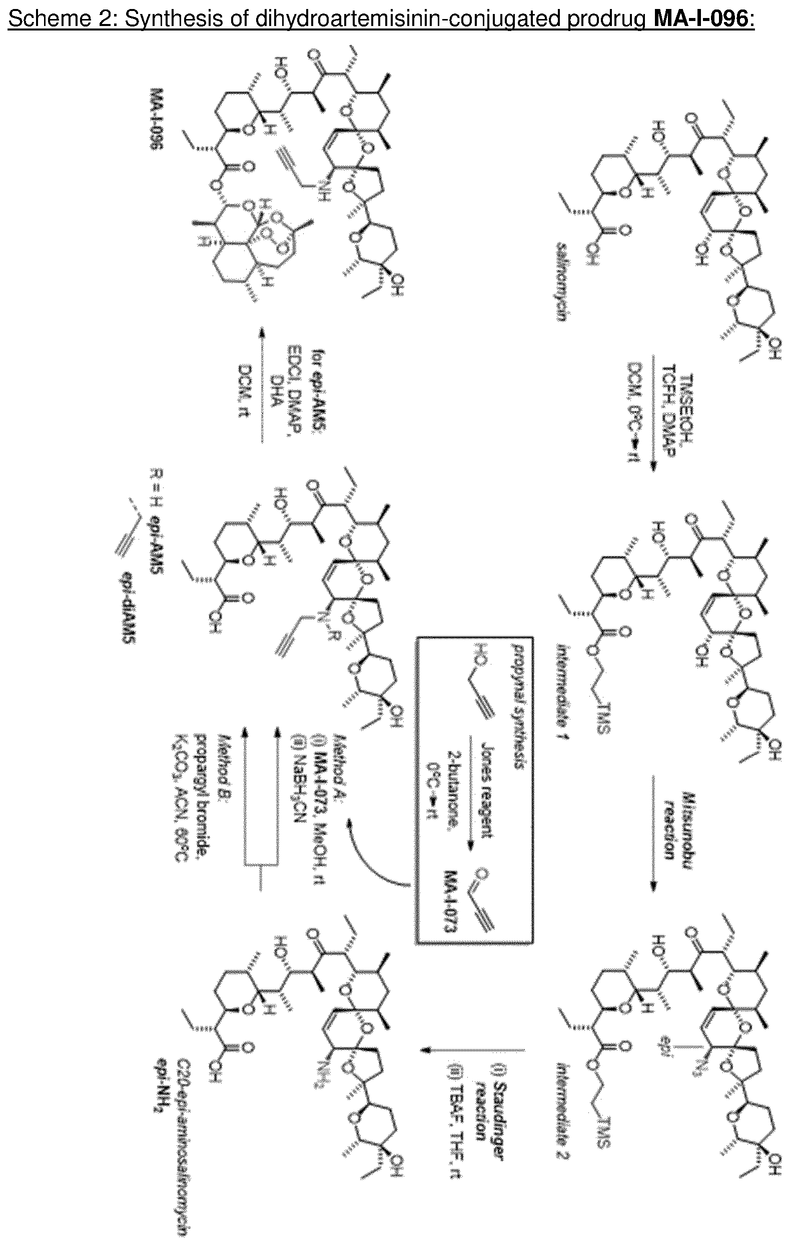

*Method A:* To a stirred solution of C20-*epi*-aminosalinomycin (50 mg, 0.07 mmol, 1.0 equiv.) in anhydrous MeOH (2 mL) at room temperature, propynal **(MA-I-073,** freshly prepared) (4.4 mg, 0.08 mmol, 1.1 equiv.) and glacial acetic acid (2 drops) were added, and the resulting mixture was stirred for 4 h. After that, NaBH₃CN (9.0 mg, 0.15 mmol, 2.0 equiv.) dissolved in anhydrous MeOH (1 mL) was introduced dropwise. The mixture was stirred overnight, then the solvent was evaporated in vacuo to dryness. The residue was purified using HPLC equipped with a C18-reverse phase column (gradient: ACN/H₂O 1/1 to ACN). Pure product of the reaction was obtained as a white amorphous solid with 25% yield.

*Method B:* To a stirred solution of C20-*epi*-aminosalinomycin (50 mg, 0.07 mmol, 1.0 equiv.) and K₂CO₃ (10 mg, 0.07 mmol, 1.0 equiv.) in anhydrous ACN (2 mL) at room temperature, propargyl bromide (80 wt % in toluene) (28 mg, 0.24 mmol, 3.5 equiv.) was added drop by drop. The reaction mixture was then heated to 60 °C, and stirred vigorously for the next 20 h. After that, the reaction mixture was filtered and the filtrate was evaporated in vacuo. The oily residue was dissolved in CH₂Cl₂ (10 mL), and extracted twice with aqueous solution of H₂SO₄ (15 mM, 2 x 7 mL) and water (7 mL). The organic layer was evaporated under reduced pressure, and purified chromatographically using HPLC equipped with a C18-reverse phase column (gradient: ACN/H₂O 1/1 to ACN). Pure product of the reaction was obtained as a white amorphous solid with 52% yield.

**C20-*epi*-ironomycin *(epi-AM5):*** Yield: 27 mg, 52%. Isolated as a white amorphous solid, >95% pure by NMR, and a single spot by TLC; R_{f}: 0.24 in CH₂Cl₂/EtOAc 50%. Strain green with PMA; ¹H NMR (500 MHz, CD₃CN) δ 6.18-6.05 (m, 2H), 4.15 (d, J = 9.8 Hz, 1H), 3.93 (dd, J = 10.4, 3.9 Hz, 1H), 3.88 (dd, J = 16.5, 1.8 Hz, 1H), 3.76 (dd, J = 13.0, 6.5 Hz, 1H), 3.63-3.56 (m, 3H), 3.50 (dd, J = 23.1, 6.2 Hz, 2H), 2.98 (td, J = 10.2, 4.2 Hz, 1H), 2.72 (td, J = 14.6, 7.2 Hz, 2H), 2.62 (d, J = 10.2 Hz, 1H), 2.52-2.41 (m, 1H), 2.25-2.12 (m, 1H), 2.10-0.60 (m, 55H) ppm; ¹³C NMR (126 MHz, CD₃CN) δ 213.5, 178.3, 132.0, 126.5, 111.7, 99.9, 88.3, 82.6, 77.7, 77.6, 75.5, 74.4, 73.0, 72.4, 71.4, 69.2, 55.9, 55.3, 49.8, 48.4, 40.1, 39.2, 36.9, 36.4, 35.2, 33.4, 31.8, 31.7, 30.3, 28.9, 26.9, 25.9, 23.7, 22.9, 20.5, 17.7, 17.6, 16.1, 15.0, 13.7, 13.2, 12.1, 11.6, 7.6, 6.7 ppm; HRMS (ESI⁺) m/z [M+H]⁺ Calcd for C₄₅H₇₄NO₁₀ 788.5313, Found 788.5310.

**C20-*epi*-dipropargyloaminosalinomycin (*epi*-diAM5):** Yield: 1.4 mg, 3%. Isolated as a white amorphous solid, >95% pure by NMR, and a single spot by TLC; R_{f}: 0.70 in CH₂Cl₂/EtOAc 50%. Strain green with PMA; ¹H NMR (500 MHz, CD₂Cl₂) δ 6.10 (dd, J = 10.4, 1.6 Hz, 1H), 5.54 (dd, J = 10.4, 2.9 Hz, 1H), 4.01 (dd, J = 8.7, 8.2 Hz, 2H), 3.78 (ddd, J = 17.5, 15.3, 6.1 Hz, 4H), 3.70-3.60 (m, 3H), 3.57 (dd, J = 11.2, 1.3 Hz, 1H), 3.46-3.40 (m, 1H), 3.14-2.92 (m, 3H), 2.85 (dd, J = 8.5, 4.3 Hz, 1H), 2.77 (dd, J = 11.3, 7.2 Hz, 1H), 2.34-2.26 (m, 2H), 2.16 (dd, J = 11.3, 8.6 Hz, 1H), 2.10-0.60 (m, 53H) ppm; ¹³C NMR (126 MHz, CD₂Cl₂) δ 217.6, 177.2, 130.9, 127.2, 109.9, 99.0, 85.3, 81.2, 78.2, 76.6, 75.3, 72.5, 72.1 (2C), 71.7, 71.3, 71.1, 60.8, 56.4, 49.6, 48.3, 41.4, 39.5, 36.8, 36.1, 34.8, 33.2, 31.0, 29.7, 29.4, 28.7, 26.4, 22.8, 22.7, 21.2, 21.0, 20.2, 18.6, 16.2, 14.6, 13.9, 13.5, 12.1, 11.5, 7.4, 6.5 ppm, two signals overlapped; HRMS (ESI⁺) m/z [M+H]⁺ Calcd for C₄₈H₇₆NO₁₀ 826.5469, Found 826.5467.

### Synthesis of dihydroartemisinin-conjugated prodrug MA-I-096 (compound of formula (I) according to the invention):

To a stirred solution of C20-*epi*-ironomycin ***epi*-AM5** (44 mg, 0.06 mmol, 1.0 equiv.) and dihydroartemisinin (32 mg, 0.11 mmol, 2.0 equiv.) in anhydrous CH₂Cl₂ (4 mL), was added EDCI hydrochloride (13 mg, 0.07 mmol, 1.2 equiv.) and DMAP (3.0 mg, 0.03 mmol, 0.5 equiv.) in one portion. The resulting mixture was stirred at room temperature for 4 days. After that, the reaction mixture was evaporated in vacuo and purified using HPLC equipped with a C18-reverse phase column (gradient: ACN/H₂O 1/1 to ACN) to give the pure product of the reaction **MA-I-096** (25% yield) as a white amorphous solid.

Yield: 15 mg, 25%. Isolated as a white amorphous solid, >95% pure by NMR, and a single spot by TLC; R_{f}: 0.74 in cyclohexane/EtOAc 50%. Strain green with PMA; ¹H NMR (500 MHz, CD₃CN) δ 6.17 (d, J = 9.7 Hz, 1H), 6.00 (dd, J = 10.5, 4.5 Hz, 1H), 5.96 (d, J = 10.6 Hz, 1H), 5.59 (s, 1H), 3.84-3.74 (m, 2H), 3.47 (q, J = 6.8 Hz, 1H), 3.36 (dd, J = 9.8, 1.8 Hz, 1H), 3.31 (dd, J = 10.4, 1.2 Hz, 1H), 3.25-3.19 (m, 3H), 2.97 (dq, J = 10.1, 7.2 Hz, 1H), 2.93 (d, J = 4.5 Hz, 1H), 2.86 (td, J = 11.1, 3.7 Hz, 1H), 2.52 (s, 1H), 2.48-2.41 (m, 2H), 2.25-2.17 (m, 2H), 2.05 (ddd, J = 14.3, 13.3, 3.8 Hz, 1H), 2.00-0.40 (m, 74H) ppm; ¹³C NMR (126 MHz, CD₃CN) δ 215.3, 175.3, 131.3, 124.1, 110.4, 104.8, 99.9, 94.4, 92.9, 88.2, 84.0, 81.4, 81.3, 77.8, 75.1, 74.1, 72.8, 72.2, 71.4, 69.4, 57.6, 56.6, 52.5, 50.6, 48.7, 46.8, 40.6, 39.7, 37.5, 37.4, 37.4, 37.1, 36.6, 35.1, 34.2, 33.8, 32.7, 31.8, 30.4, 28.9, 26.8, 26.2, 25.4, 25.2, 23.0, 22.5, 22.4, 21.1, 20.4, 20.1, 17.6, 17.0, 15.1, 14.5, 13.8, 13.0, 12.7, 11.2, 7.7, 6.8 ppm; HRMS (ESI⁺) m/z [M+H]⁺ Calcd for C₆₀H₉₆NO₁₄ 1054.6831, Found 1054.6841.

### 3/ Synthesis of dihydroartemisinin-conjugated prodrugs of narasin

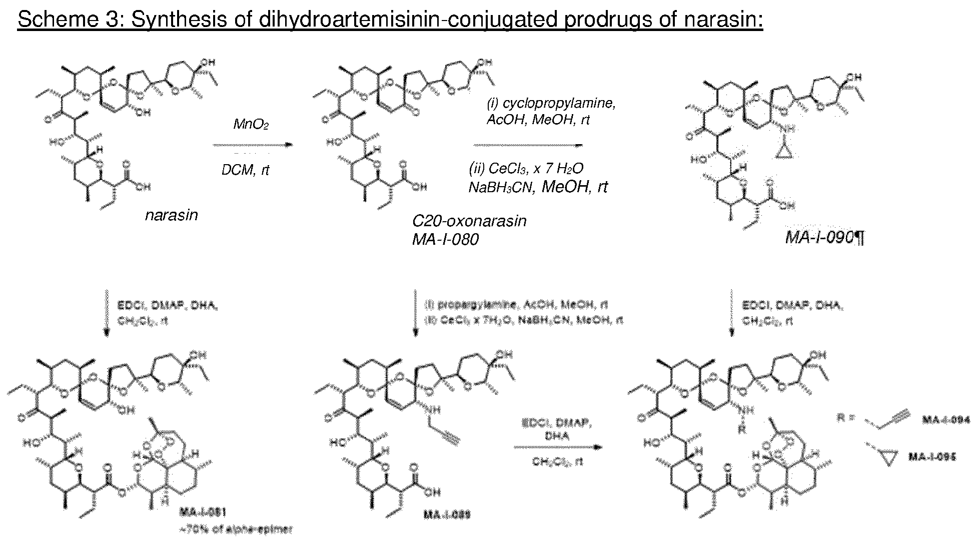

**Synthesis of C20-oxonarasin MA-I-080:** To a stirred solution of narasin (40 mg, 0.05 mmol, 1.0 equiv.) in CH₂Cl₂ (5 mL), activated MnO₂ (174 mg, 2.00 mmol, 40.0 equiv.) was added in one portion, and the black suspension was stirred at room temperature for 24 h. After that, the second portion of activated MnO₂ (174 mg, 2.00 mmol, 40.0 equiv.) was added, and the stirring was continued at room temperature for the next 24 h. The reaction mixture was then filtered through Celite, evaporated to dryness under reduced pressure, and the residue was purified using HPLC equipped with a C18-reverse phase column (gradient: ACN/H₂O/FA 4/1/0.1 to ACN/FA 1/0.1). Pure product of the reaction was quantitatively obtained as a white amorphous solid.

Yield: 39 mg, 98%. Isolated as a white amorphous solid, >95% pure by NMR, and a single spot by TLC; R_{f}: 0.44 in CH₂Cl₂/EtOAc 50%. UV-active and strain green with PMA; ¹H NMR (500 MHz, CD₂Cl₂) δ 7.16 (d, J = 10.8 Hz, 1H), 6.10 (d, J = 10.8 Hz, 1H), 4.12 (q, J = 6.1 Hz, 1H), 3.91 (d, J = 10.3 Hz, 1H), 3.86 (dd, J = 10.3, 5.3 Hz, 1H), 3.78 (d, J = 10.2 Hz, 1H), 3.51 (dd, J = 9.8, 1.7 Hz, 1H), 3.44 (t, J = 4.8 Hz, 1H), 3.21 (s, 1H), 2.85-2.69 (m, 2H), 2.59 (dd, J = 10.9, 1.4 Hz, 1H), 2.45 (td, J = 12.4, 8.9 Hz, 1H), 2.35-2.20 (m, 1H), 2.05-0.60 (m, 55H) ppm; ¹³C NMR (126 MHz, CD₂Cl₂) δ 214.5, 191.1, 177.5, 144.6, 126.4, 106.4, 98.1, 91.6, 78.7, 76.3, 75.3, 74.8, 73.1, 72.4, 68.9, 55.4, 49.7, 48.8, 40.4, 39.2, 36.7, 36.1, 35.8, 34.7, 33.1, 31.0, 29.5, 28.6, 25.0, 24.3, 23.9, 22.0, 19.1, 18.0, 16.13, 16.07, 14.0, 13.7, 13.0, 12.3, 12.2, 7.5, 6.9 ppm; HRMS (ESI⁺) m/z [M+NH₄]⁺ Calcd for C₄₃H₇₄NO₁₁ 780.5262, Found 780.5265.

**Synthesis of narasin-dihydroartemisinin prodrug MA-I-081 (compound of formula (I) according to the invention):** To a stirred solution of narasin (40 mg, 0.05 mmol, 1.0 equiv.) and dihydroartemisinin (28 mg, 0.10 mmol, 2.0 equiv.) in anhydrous CH₂Cl₂ (2 mL), was added EDCI hydrochloride (12 mg, 0.06 mmol, 1.2 equiv.) and DMAP (3.0 mg, 0.02 mmol, 0.5 equiv.). The resulting mixture was stirred at room temperature for 4 days. After that, the reaction mixture was evaporated to dryness under reduced pressure, and the residue was purified using HPLC equipped with a C18-reverse phase column (gradient: ACN/H₂O/FA 4/1/0.1 to ACN/FA 1/0.1). Product of the reaction was obtained as a white amorphous solid with 13% yield.

Yield: 6.8 mg, 13%. Isolated as a white amorphous solid, >95% pure by NMR, and two spots by TLC (~70% of α-epimer); R_{f}: 0.73 and 0.89 in CH₂Cl₂/EtOAc 50%. Strain green with PMA; ¹H NMR (500 MHz, CD₂Cl₂) δ 6.30 (dd, J = 9.3, 5.8 Hz, 2H), 6.02 (dd, J = 10.7, 1.7 Hz, 1H), 5.93 (td, J = 10.9, 2.1 Hz, 2H), 5.83 (s, 1H), 5.73 (s, 1H), 5.59 (dd, J = 9.9, 1.5 Hz, 1H), 4.10 (td, J = 9.8, 5.5 Hz, 2H), 3.98 (dd, J = 20.6, 11.1 Hz, 3H), 3.86-3.71 (m, 3H), 3.67 (dd, J = 11.4, 2.0 Hz, 1H), 3.64-3.50 (m, 4H), 3.19 (dq, J = 10.1, 7.1 Hz, 1H), 3.02 (td, J = 10.9, 4.0 Hz, 1H), 2.95 (td, J = 10.6, 3.4 Hz, 1H), 2.91-2.85 (m, 1H), 2.82 (d, J = 5.4 Hz, 1H), 2.74-2.60 (m, 3H), 2.55-2.44 (m, 2H), 2.44-2.12 (m, 8H), 2.10-0.60 (m, 149H) ppm; ¹³C NMR (126 MHz, CD₂Cl₂) δ 214.4, 213.9, 174.7, 174.5, 133.5, 133.1, 130.7, 121.1, 109.6, 106.8, 104.34, 104.26, 99.4, 97.8, 94.2, 93.8, 92.2, 92.1, 88.2, 86.5, 81.4, 80.9, 80.8, 78.3, 77.8, 77.6, 75.8, 74.5, 73.7, 73.0, 72.4, 71.10, 71.08, 69.2, 68.4, 67.9, 67.4, 57.1, 55.2, 52.1, 52.0, 50.00, 49.96, 48.7, 48.3, 46.43, 46.35, 40.7, 39.4, 39.2, 37.1, 37.0, 36.81, 36.79, 36.77, 36.7, 36.55, 36.53, 36.4, 36.0, 35.9, 34.8, 33.6, 33.5, 33.4, 33.2, 31.6, 31.1, 31.0, 30.6, 29.7, 29.6, 29.5, 28.7, 26.0, 25.9, 25.1, 25.03, 24.97, 22.6, 22.4, 22.0, 21.8, 20.7, 20.24, 20.18, 19.2, 19.1, 18.7, 18.2, 17.4, 16.4, 15.7, 14.80, 14.75, 14.2, 13.8, 13.6, 13.4, 13.0, 12.82, 12.77, 12.64, 12.62, 12.58, 8.1, 7.9, 6.5 ppm; HRMS (ESI⁺) m/z [M+NH₄]⁺ Calcd for C₅₈H₉₈NO₁₅ 1048.6936, Found 1048.6945.

**Synthesis of C20-propargyloaminonarasin MA-I-089:** Under argon atmosphere, C20-oxonarasin **(MA-I-080)** (67 mg, 0.09 mmol, 1.0 equiv.) was dissolved in anhydrous MeOH (4 mL), followed by the addition of molecular sieves 4Å, propargylamine (55 µL, 0.87 mmol, 10.0 equiv.) and glacial acetic acid (35 µL). The mixture was stirred 3 h at room temperature, prior to the addition of CeCl₃ x 7H₂O (32 mg, 0.09 mmol, 1.0 equiv.), and the dropwise addition of a solution of NaBH₃CN (7.0 mg, 0.12 mmol, 1.3 equiv.) in anhydrous MeOH (3 mL) over 8 h at room temperature, using a syringe pump. Then, the yellow reaction mixture was evaporated to dryness. A small portion of CH₂Cl₂ was added to the oily residue, and the insoluble components were filtered off. The filtrate was evaporated in vacuo and purified using HPLC equipped with a C18-reverse phase column (gradient: ACN/H₂O/FA 1/1/0.1 to ACN/FA 1/0.1). Pure product of the reaction was obtained as a white amorphous solid with 39% yield.

Yield: 27 mg, 39%. Isolated as a white amorphous solid, >95% pure by NMR, and a single spot by TLC; R_{f}: 0.78 in CH₂Cl₂/EtOAc 50%. Strain green with PMA; ¹H NMR (400 MHz, CD₂Cl₂) δ 6.37 (dd, J = 10.0, 5.3 Hz, 1H), 6.20 (d, J = 10.2 Hz, 1H), 4.24 (d, J = 9.8 Hz, 1H), 4.16 (dt, J = 16.0, 2.1 Hz, 1H), 3.89-3.79 (m, 2H), 3.78-3.70 (m, 2H), 3.59 (d, J = 5.4 Hz, 1H), 3.57-3.50 (m, 2H), 2.67-2.54 (m, 3H), 2.29 (t, J = 2.3 Hz, 1H), 2.25-2.14 (m, 1H), 2.10-2.00 (m, 1H), 1.99-1.83 (m, 4H), 1.80-0.60 (m, 53H) ppm; ¹³C NMR (101 MHz, CD₂Cl₂) δ 215.0, 178.7, 130.6, 128.0, 109.0, 99.7, 88.7, 80.3, 78.2, 77.6, 75.7, 73.8, 73.5, 73.4, 71.2, 69.2, 55.9, 53.2, 50.4, 50.3, 40.0, 39.5, 38.5, 36.5, 36.1, 35.6, 32.8, 31.9, 30.9, 29.6, 29.2, 28.8, 24.4 (2C), 21.8, 17.9, 17.6, 16.5, 15.8, 14.6, 13.5, 13.2, 12.9, 12.4, 8.2, 6.6 ppm; HRMS (ESI⁺) m/z [M+H]⁺ Calcd for C₄₆H₇₆NO₁₀ 802.5469, Found 802.5469.

**Synthesis of C20-cyclopropyloaminonarasin MA-I-090:** Under argon atmosphere, C20-oxonarasin **(MA-I-080)** (88 mg, 0.11 mmol, 1.0 equiv.) was dissolved in anhydrous MeOH (5 mL), followed by the addition of molecular sieves 4Å, cyclopropylamine (79 µL, 1.11 mmol, 10.0 equiv.) and glacial acetic acid (45 µL). The mixture was stirred 3 h at room temperature, prior to the addition of CeCl₃ x 7H₂O (42 mg, 0.11 mmol, 1.0 equiv.), and the dropwise addition of a solution of NaBH₃CN (9.0 mg, 0.16 mmol, 1.4 equiv.) in anhydrous MeOH (4 mL) over 8 h at room temperature, using a syringe pump. After that, the cloudy reaction mixture was filtered, and the filtrate was evaporated to dryness. The oily residue was dissolved in a small portion of CH₂Cl₂, the insoluble components were filtered off, the filtrate was evaporated in vacuo and purified using HPLC equipped with a C18-reverse phase column (gradient: ACN/H₂O/FA 1/1/0.1 to ACN/FA 1/0.1). Pure product of the reaction was obtained as a white amorphous solid with 47% yield.

Yield: 44 mg, 47%. Isolated as a white amorphous solid, >95% pure by NMR, and a single spot by TLC; R_{f}: 0.87 in CH₂Cl₂/EtOAc 50%. Strain green with PMA; ¹H NMR (500 MHz, CD₂Cl₂) δ 6.43 (dd, J = 10.3, 4.7 Hz, 1H), 6.17 (d, J = 10.3 Hz, 1H), 4.20 (dd, J = 10.0, 1.3 Hz, 1H), 3.83-3.75 (m, 2H), 3.73 (q, J = 6.8 Hz, 1H), 3.56 (ddd, J = 13.8, 10.2, 2.9 Hz, 2H), 3.36 (d, J = 4.7 Hz, 1H), 2.80 (ddd, J = 11.0, 7.4, 3.8 Hz, 1H), 2.64 (dq, J = 9.9, 7.1 Hz, 1H), 2.57 (ddd, J = 9.3, 7.7, 5.0 Hz, 2H), 2.22-2.12 (m, 1H), 2.09-1.86 (m, 5H), 1.80-0.65 (m, 55H), 0.62-0.55 (m, 1H), 0.52-0.45 (m, 1H) ppm; ¹³C NMR (126 MHz, CD₂Cl₂) δ 215.0, 178.6, 130.8, 126.8, 108.2, 99.9, 89.1, 78.1, 77.4, 76.0, 73.7, 71.2, 69.4, 56.9, 56.0, 50.4 (2C), 40.3, 39.4, 38.7, 36.1, 35.6, 32.9, 31.3, 30.9, 30.6, 29.5, 29.2, 28.8, 25.0, 24.6, 22.2, 17.9, 17.7, 16.6, 15.9, 14.5, 13.6, 13.4, 13.1, 12.5, 8.2, 6.6, 6.2, 5.1 ppm, one signal overlapped; HRMS (ESI⁺) m/z [M+H]⁺ Calcd for C₄₆H₇₈NO₁₀ 804.5626, Found 804.5626.

### General procedure for the synthesis of dihydroartemisinin-conjugated prodrugs of the invention:

To a stirred solution of respective C20-amine derivative of narasin (1.0 equiv.) and dihydroartemisinin (2.0 equiv.) in anhydrous CH₂Cl₂, was added EDCI hydrochloride (1.2 equiv.) and DMAP (0.5 equiv.) in one portion. The resulting mixture was stirred at room temperature for 4 days. After that, the reaction mixture was evaporated in vacuo and purified using HPLC equipped with a C18-reverse phase column (gradient: ACN/H₂O/FA 1/1/0.1 to ACN/FA 1/0.1) to give the pure products of the reaction (16-18% yield) as white amorphous solids.

**C20-propargyloaminonarasin-dihydroartemisinin prodrug MA-I-094 (compound of formula (I) according to the invention):** Yield: 8.6 mg, 16%. Isolated as a white amorphous solid, >95% pure by NMR, and a single spot by TLC; R_{f}: 0.66 in cyclohexane/EtOAc 50%. Strain green with PMA; ¹H NMR (400 MHz, CD₂Cl₂) δ 6.31 (d, J = 9.7 Hz, 1H), 6.06 (dd, J = 10.7, 2.0 Hz, 1H), 6.01 (dd, J = 10.8, 0.7 Hz, 1H), 5.74 (s, 1H), 4.10 (dd, J = 10.6, 5.2 Hz, 1H), 4.00 (d, J = 10.1 Hz, 1H), 3.77 (q, J = 6.7 Hz, 1H), 3.64-3.55 (m, 2H), 3.49 (ddd, J = 8.4, 7.0, 2.4 Hz, 3H), 3.22 (ddd, J = 15.3, 10.5, 7.5 Hz, 1H), 3.18-3.12 (m, 1H), 2.98 (td, J = 10.6, 3.3 Hz, 1H), 2.75-2.68 (m, 1H), 2.55-2.45 (m, 1H), 2.40-0.60 (m, 80H) ppm; ¹³C NMR (101 MHz, CD₂Cl₂) δ 214.5, 174.5, 131.7, 120.9, 106.9, 104.3, 99.0, 93.8, 92.1, 87.8, 82.9, 81.8, 80.8, 77.9, 77.5, 74.1, 72.8, 71.14, 71.06, 69.0, 57.3, 55.8, 52.1, 50.0, 48.6, 46.4, 40.8, 39.3, 37.6, 37.1, 36.9, 36.8, 36.5, 36.0, 34.8, 33.5, 33.0, 31.4, 30.9, 29.65, 29.60, 28.7, 25.9, 25.5, 25.0, 22.6, 22.4, 21.9, 21.5, 20.2, 18.9, 17.4, 15.7, 14.7, 14.1, 13.7, 12.81, 12.77, 12.6, 8.0, 6.6 ppm; HRMS (ESI⁺) m/z [M+H]⁺ Calcd for C₆₁H₉₈NO₁₄ 1068.6987, Found 1068.6982.

**C20-cyclopropylaminonarasin-dihydroartemisinin prodrug MA-I-095 (compound of formula (I) according to the invention):** Yield: 9.4 mg, 18%. Isolated as a white amorphous solid, >95% pure by NMR, and a single spot by TLC; R_{f}: 0.74 in cyclohexane/EtOAc 50%. Strain green with PMA; ¹H NMR (500 MHz, CD₂Cl₂) δ 6.30 (d, J = 9.7 Hz, 1H), 6.05 (s, 2H), 5.74 (s, 1H), 4.10 (dd, J = 10.5, 5.1 Hz, 1H), 3.99 (dd, J = 10.2, 0.9 Hz, 1H), 3.72 (q, J = 6.8 Hz, 1H), 3.61 (dd, J = 9.6, 2.7 Hz, 1H), 3.54-3.45 (m, 2H), 3.28-3.14 (m, 2H), 2.97 (td, J = 10.7, 3.6 Hz, 1H), 2.75-2.68 (m, 1H), 2.54-2.46 (m, 1H), 2.38-2.25 (m, 4H), 2.20-0.60 (m, 76H), 0.47-0.35 (m, 3H), 0.29 (d, J = 6.4 Hz, 1H) ppm; ¹³C NMR (126 MHz, CD₂Cl₂) δ 214.5, 174.5, 132.5, 120.8, 107.2, 104.3, 98.9, 93.8, 92.2, 87.5, 81.8, 80.8, 77.9, 77.5, 73.4, 72.9, 71.1, 69.1, 57.3, 56.6, 52.1, 50.0, 48.6, 46.4, 40.8, 39.3, 37.1, 36.8, 36.43, 36.36, 36.0, 34.8, 33.5, 33.1, 31.9, 30.9, 29.7, 29.6, 29.3, 28.8, 25.9, 25.0, 24.9, 22.6, 22.1, 21.9, 21.5, 20.2, 18.8, 17.4, 15.8, 14.5, 14.1, 13.7, 12.9, 12.8, 12.6, 8.1, 7.2, 6.6, 6.5 ppm; HRMS (ESI⁺) m/z [M+H]⁺ Calcd for C₆₁H₁₀₀NO₁₄ 1070.7144, Found 1070.7152.

### 4/ Synthesis of compound AM9 (negative control)

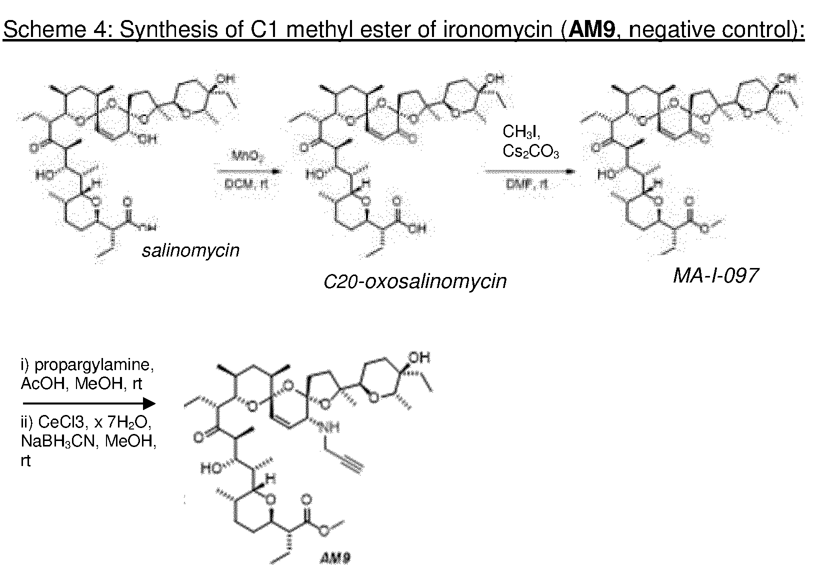

### Synthesis of C1 methyl ester of C20-oxosalinomycin MA-I-097:

Under argon atmosphere, C20-oxosalinomycin (200 mg, 0.26 mmol, 1.0 equiv.) was dissolved in anhydrous DMF (5 mL). Then, cesium carbonate (111 mg, 0.34 mmol, 1.3 equiv.) was added, followed by the addition of methyl iodide (21 µL, 0.34 mmol, 1.3 equiv.) in one portion. The reaction mixture was stirred at room temperature for 24 h. After that, the yellow reaction mixture was evaporated to dryness under reduced pressure. CH₂Cl₂ was added to the oily residue, the insoluble components were filtered off, the filtrate was evaporated in vacuo, and purified chromatographically using HPLC equipped with a C18-reverse phase column (gradient: ACN to ACN/H₂O 5/95). Pure product of the reaction was obtained as a white amorphous solid with 79% yield.

Yield: 161 mg, 79%. Isolated as a white amorphous solid, >95% pure by NMR, and a single spot by TLC; R_{f}: 0.87 in cyclohexane/EtOAc 50%. UV-active and strain green with PMA; ¹H NMR (500 MHz, CD₂Cl₂) δ 7.24 (d, J = 10.8 Hz, 1H), 6.20 (d, J = 10.8 Hz, 1H), 3.94 (dd, J = 10.9, 5.7 Hz, 1H), 3.90 (dd, J = 10.1, 0.9 Hz, 1H), 3.75-3.67 (m, 5H), 3.55 (dd, J = 9.9, 1.8 Hz, 1H), 3.38-3.32 (m, 1H), 3.00 (td, J = 10.9, 4.5 Hz, 1H), 2.91 (dq, J = 10.0, 7.1 Hz, 1H), 2.65 (dt, J = 8.7, 3.3 Hz, 1H), 2.60-2.51 (m, 1H), 2.13-1.99 (m, 2H), 1.95-0.60 (m, 53H) ppm; ¹³C NMR (126 MHz, CD₂Cl₂) δ 214.1, 190.7, 176.7, 144.2, 127.5, 105.8, 98.0, 89.0, 78.4, 77.5, 75.3, 72.7, 72.0, 71.1, 70.1, 57.1, 52.6, 49.2, 47.6, 40.3, 39.3, 36.8, 34.7, 34.5, 34.3, 30.8, 29.7, 28.5, 26.6, 23.2, 23.0, 21.2, 20.1, 18.6, 17.8, 16.0, 14.6, 14.1, 12.5, 12.1, 11.2, 7.4, 6.6 ppm; HRMS (ESI⁺) m/z [M+NH₄]⁺ Calcd for C₄₃H₇₄NO₁₁ 780.5262, Found 780.5263.

### Synthesis of C1 methyl ester of ironomycin AM9:

C1 methyl ester of C20-oxosalinomycin **(MA-I-097)** (100 mg, 0.13 mmol, 1.0 equiv.) was dissolved in anhydrous MeOH (5 mL), followed by the addition of propargylamine (84 µL, 1.30 mmol, 10.0 equiv.) and glacial acetic acid (50 µL). The solution was stirred 3 h at room temperature, prior to the addition of CeCl₃ x 7H₂O (48 mg, 0.13 mmol, 1.0 equiv.) and the dropwise addition of a solution of NaBH₃CN (11 mg, 0.18 mmol, 1.4 equiv.) in 2 mL of anhydrous MeOH over 8 h at room temperature, using a syringe pump. After that, the cloudy reaction mixture was filtered and the filtrate was evaporated to dryness. Then, CH₂Cl₂ was added to the oily residue, the insoluble components were filtered off, the filtrate was evaporated in vacuo, and purified chromatographically using HPLC equipped with a C18-reverse phase column (gradient: ACN/H₂O/FA 1/1/0.1 to ACN/FA 1/0.1). Pure product of the reaction was obtained as a white amorphous solid with 29% yield.

Yield: 31 mg, 29%. Isolated as a white amorphous solid, >95% pure by NMR, and a single spot by TLC; R_{f}: 0.69 in cyclohexane/EtOAc 50%. Strain green with PMA; ¹H NMR (500 MHz, CD₂Cl₂) δ 6.14 (d, J = 10.2 Hz, 1H), 6.04 (d, J = 10.5 Hz, 1H), 3.99-3.94 (m, 2H), 3.84-3.77 (m, 4H), 3.66-3.52 (m, 5H), 3.28 (s, 1H), 3.09-2.98 (m, 2H), 2.71- 2.67 (m, 1H), 2.32-2.24 (m, 2H), 2.18-2.12 (m, 1H), 2.00-0.60 (m, 55H) ppm; ¹³C NMR (126 MHz, CD₂Cl₂) δ 214.9, 176.5, 130.7, 122.5, 106.6, 99.0, 88.0, 82.0, 80.0, 77.5, 75.3, 74.1, 72.1, 71.9, 71.1, 69.7, 56.9, 55.8, 52.6, 49.0, 48.3, 40.7, 39.1, 37.5, 37.3, 36.8, 33.3, 31.2, 31.0, 29.6, 28.6, 26.6, 25.4, 23.1, 22.4, 20.19, 20.16, 17.6, 15.7, 14.8, 14.0, 13.4, 12.0, 11.3, 7.6, 6.6 ppm; HRMS (ESI⁺) m/z [M+H]⁺ Calcd for C₄₆H₇₆NO₁₀ 802.5469, Found 802.5459.

### Cell viability

Cell viability was evaluated by plating 4000 cells/well in 96-well plates using CellTiter-Blue viability assay according to the manufacturer's protocol. Cells were treated with different concentrations of ironomycin as indicated for 72 h. CellTiter-Blue reagent (Promega, G8081) was added after 72 h treatment and cells were incubated for 2 h before recording fluorescence intensities (ex. 560 nm; em. 590 nm) using a Perkin Elmer Wallac 1420 Victor2 Microplate Reader.

IC₅₀ were also measured.

The results are in the table below and in Figure 1.

### IC₅₀ values in µM of Ironomycin/Narasin derivatives and prodrugs of the invention on pancreatic ductal adenocarcinoma cell lines

| **Cell line** | **PDAC030T** | **PDAC053T** | **PDAC085T** | **PDAC090T** | **PDAC211T** |
|---|---|---|---|---|---|
| Salinomycin (SalH) | 1.61 | 4.50 | 1.56 | 2.66 | 1.29 |
| Sal-DHA prodrug (MA-I-023) | 0.75 | 3.88 | 3.32 | 4.53 | 3.13 |
| Ironomycin (AM5) | 2.67 | 2.60 | 0.72 | 0.63 | 0.18 |
| Ironomycin-DHA prodrug (MA-I-062) | 0.50 | 1.79 | 1.17 | 2.19 | 0.10 |
| AM9 (negative control) | 5.67 | 7.46 | 8.88 | 5.89 | 5.01 |
| AM23 | 0.11 | 0.83 | 0.36 | 0.21 | 0.04 |
| AM23-DHA prodrug (MA-I-082) | 0.41 | 1.26 | 0.74 | 0.80 | 0.24 |
| Narasin | 1.18 | 2.00 | 0.50 | 1.16 | 0.73 |
| Narasin-DHA prodrug (MA-I-081) | 0.09 | 0.54 | 0.44 | 0.74 | 0.45 |
| Nar-AM5 | 0.22 | 0.88 | 0.12 | 0.80 | 0.22 |
| Nar-AM5-DHA prodrug (MA-I-094) | 0.18 | 0.87 | 0.58 | 1.94 | 0.30 |
| Nar-AM23 | 0.06 | 0.20 | 0.04 | 0.32 | 0.05 |
| Nar-AM23-DHA prodrug (MA-I-095) | 0.12 | 0.09 | 0.07 | 0.18 | 0.49 |
| Epi-AM5 | 2.87 | 2.17 | 0.67 | 4.16 | 1.48 |
| Epi-AM5-DHA prodrug (MA-I-096) | 0.09 | 0.52 | 0.36 | 0.69 | 0.33 |
| DHA | 8.08 | >50.00 | 21.55 | 35.37 | 10.29 |

In conclusion, the prodrugs of the invention show comparable efficacy against cancer cells compared to parent drugs, with some level of specificity against cancerous cells.

### IC₅₀ values in µM of Ironomycin/Narasin derivatives and prodrugs of the invention on breast cancer cell lines

In addition to the above distinct patient-derived pancreatic ductal adenocarcinoma (PDAC) cell lines, the compounds were also evaluated against nontumorigenic epithelial breast cell MCF10A.

The inventors also evaluated the biological activity of prodrugs and parent structures against transformed human mammary epithelial HMLER CD44^{high}/CD24^{low} cells, a model of human breast cancer stem cells stable in the mesenchymal state, and against control epithelial HMLER CD44^{low}/CD24^{high} cells.

| **Cell line** | **MCF10A** | **HMLER CD44^{high}/CD24^{low}** | **HMLER CD44^{low}/CD24^{high}** |
|---|---|---|---|
| Salinomycin (SalH) | 1.13 | 1.38 | 4.36 |
| Sal-DHA prodrug (MA-I-023) | NT | 0.48 | 0.82 |
| Ironomycin (AM5) | 0.12 | 0.16 | 1.79 |
| Ironomycin-DHA prodrug (MA-I-062) | 2.03 | 0.16 | 0.48 |
| AM9 | 12.25 | 13.20 | 15.20 |
| AM23 | 0.30 | 0.01 | 0.50 |
| AM23-DHA prodrug (MA-I-082) | 3.26 | 0.18 | 0.67 |
| Narasin | 0.79 | 0.45 | 2.96 |
| Narasin-DHA prodrug (MA-I-081) | NT | 0.19 | 0.50 |
| Nar-AM5 | 0.22 | 0.11 | 1.08 |
| Nar-AM5-DHA prodrug (MA-I-094) | 3.56 | 0.54 | 1.17 |
| Nar-AM23 | 0.10 | 0.01 | 0.45 |
| Nar-AM23-DHA prodrug (MA-I-095) | 5.24 | 0.76 | 1.96 |
| Epi-AM5 | 1.34 | 1.80 | 11.80 |
| Epi-AM5-DHA prodrug (MA-I-096) | 1.37 | 0.20 | 0.36 |
| DHA | 5.45 | 2.85 | 14.16 |

It was thus found that prodrugs MA-I-062, MA-I-082, MA-I-094, and MA-I-065 were less toxic toward nontumorigenic epithelial cells than their respective parent compounds, validating the notion that using iron-cleavable prodrugs can widen the therapeutic window. Importantly, prodrugs were found to be more effective than the parent compounds in some patient-derived pancreatic cancer cells. For example, MA-I-095 was more potent than Nar-AM23 in PDAC053T cells.

## Claims

1. Compound chosen from compounds of formula (I) and their diastereoisomers: 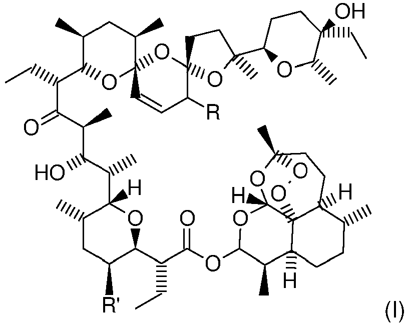
wherein R is a hydroxyl group or a group -NR1R2 where R1 and R2 are identical or different and independently chosen from H, a C1-C20 alkyl group, a C3-C6 cycloalkyl group, an alkyne group and an aralkyl group, and
wherein R' is H or methyl,
preferably R1 and R2 are different and independently chosen from H, a C1-C20 alkyl group, a C3-C6 cycloalkyl group, an alkyne group and an aralkyl group.

2. Compound according to claim 1, wherein it is chosen from compounds of formula (I') and their diastereoisomers: 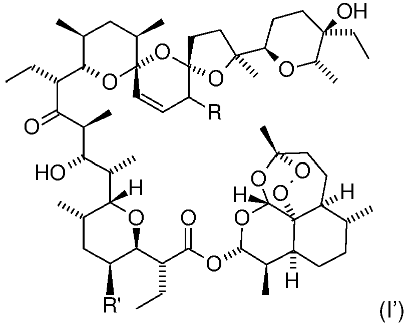 wherein R and R' are as defined above.

3. Compound according to any one of the preceding claims, wherein the C3-C6 cycloalkyl is a cyclic hydrocarbon group comprising from 3 to 6 carbon atoms, preferably it is chosen from cyclopropyl, cyclobutyl, cyclopentyl, and cyclohexyl groups, more preferably the C3-C6 cycloalkyl is cyclopropyl, and/or the alkyne group is an unsaturated hydrocarbon comprising at least one carbon-carbon triple bond (-C≡C-), preferably the alkyne group is linear, preferably the alkyne group comprises 2 to 10 carbon atoms, preferably 2 to 6 carbon atoms, preferably 3 carbon atoms, preferably the alkyne group is propynyl.

4. Compound according to any one of the preceding claims, wherein the C1-C20 alkyl group is a linear or hydrocarbon group comprising from 1 to 20 carbon atoms, preferably 2 to 15 carbon atoms, or a branched hydrocarbon group comprising from 3 to 20 carbon atoms, preferably 3 to 10 carbon atoms, and preferably ethyl, n-propyl, n-butyl, isobutyl, n-pentyl, n-hexyl or dodecyl groups.

5. Compound according to any one of the preceding claims, wherein the aralkyl is a group -(CH₂)n-aryl, wherein n is an integer from 1 to 5, and aryl is a monocyclic or polycyclic aromatic hydrocarbon group, which may be optionally substituted by at least one group chosen from a hydroxyl group, a C1-C20 alkyl group and a halogen, preferably the aralkyl group is a benzyl which is not substituted, or substituted, preferably in para position, by at least one group chosen from a hydroxyl group, a C1-C6 alkyl group such as a methyl, and a halogen such as F or Cl.

6. Compound according to any one of the preceding claims, wherein R is a hydroxyl group or a group -NR1R2, where R1 is chosen from a C1-C20 alkyl group, a C3-C6 cycloalkyl group, an alkyne group and an aralkyl group, and R2 is chosen from H, a C1-C20 alkyl group and an alkyne group; preferably R is a hydroxyl group or a group -NR1R2 where R1 and R2 are different and independently chosen from H, a C3-C6 cycloalkyl group and an alkyne group.

7. Compound according to claim 1, wherein R is a hydroxyl group.

8. Compound according to any one of claims 1 to 6, wherein R is -NR1R2, and R2 is H and R1 is a C3-C6 cycloalkyl group or an alkyne group, preferably a C2-C6 alkyne group, preferably R2 is H and R1 is cyclopropyl or propynyl.

9. Compound according to any one of the preceding claims, wherein it is chosen from the following compounds: 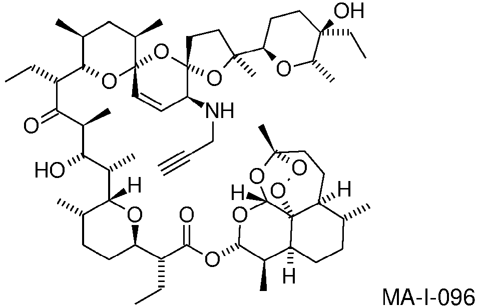 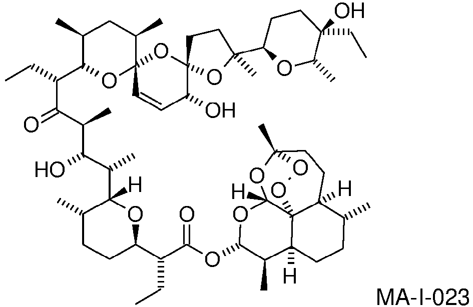 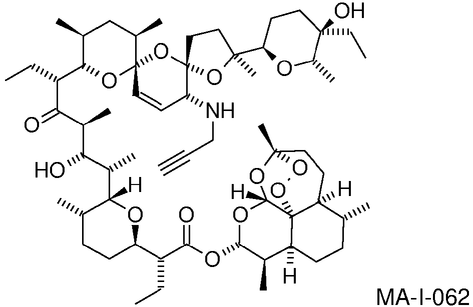 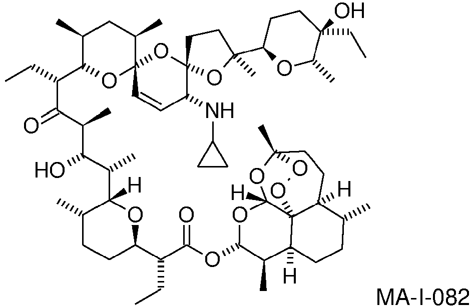 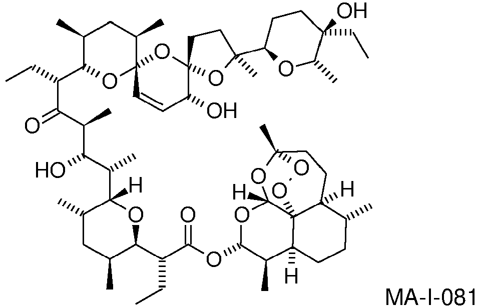 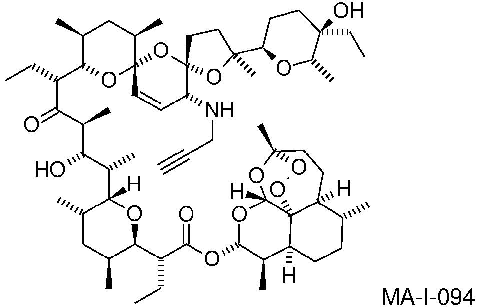 and

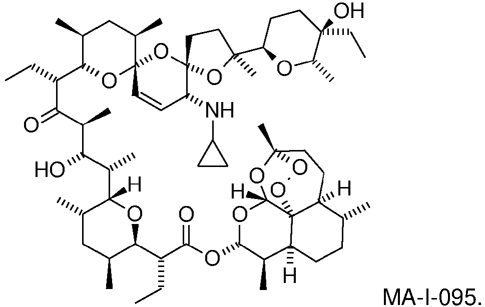

10. Composition comprising, in a pharmaceutically acceptable medium, at least one compound of formula (I) according to any one of the preceding claims.

11. Compound according to any one of claims 1 to 9, for use as a medicament.

12. Compound according to any one of claims 1 to 9, for use for preventing and/or treating cancer, and/or for preventing cancer metastasis and/or for preventing cancer recurrence and/or for decreasing resistance to a chemotherapy in a subject, preferably wherein the cancer is selected from solid and non-solid cancers, preferably from a colon cancer, a colorectal cancer such as colorectal cancers with a BRAF mutation especially BRAF V600E, a melanoma, a bone cancer, a breast cancer such as triple-negative breast cancer, a thyroid cancer, a prostate cancer, an ovarian cancer, a lung cancer, a pancreatic cancer, a glioma such as a glioblastoma, a cervical cancer, an endometrial cancer, a head and neck cancer, a liver cancer, a bladder cancer, a renal cancer, a skin cancer, a stomach cancer, a testis cancer, an urothelial cancer or an adrenocortical carcinoma, leukemia such as acute myeloid leukemia, lymphoma and multiple myeloma.

13. A product comprising:
a) a compound according to any one of claims 1 to 9, and
b) at least one additional therapy,
as combination product for a simultaneous, separate or sequential use for treating cancer, and/or for preventing cancer metastasis, and/or for preventing cancer recurrence, and/or for decreasing resistance to the additional therapy b), in a subject.

14. The product according to claim 13, wherein said additional therapy b) is immunotherapy, chemotherapy and/or radiotherapy.

15. The product according to claim 13 or 14, wherein the subject is a human suffering from a cancer and resistant to chemotherapy.

## Patentansprüche

1. Verbindung ausgewählt aus Verbindungen der Formel (I) und deren Diastereomeren: 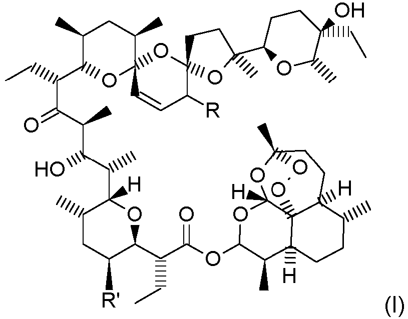
worin R eine Hydroxylgruppe oder eine Gruppe -NR¹R² ist, wobei R¹ und R² gleich oder verschieden sind und jeweils unabhängig aus H, einer C1-C20-Alkylgruppe, einer C3-C6-Cycloalkylgruppe, einer Alkingruppe oder einer Aralkylgruppe ausgewählt sind, und
worin R' H oder Methyl ist,
vorzugsweise sind R¹ und R² verschieden und jeweils unabhängig aus H, einer C1-C20-Alkylgruppe, einer C3-C6-Cycloalkylgruppe, einer Alkingruppe und einer Aralkylgruppe ausgewählt.

2. Verbindung nach Anspruch 1, wobei sie aus Verbindungen der Formel (I') und deren Diastereomeren ausgewählt ist: 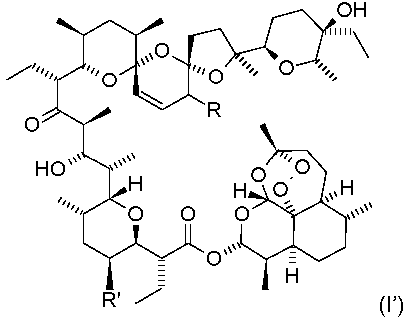 worin R und R' wie oben definiert sind.

3. Verbindung nach einem der vorhergehenden Ansprüche, wobei die C3-C6-Cycloalkylgruppe eine cyclische Kohlenwasserstoffgruppe mit 3 bis 6 Kohlenstoffatomen ist, vorzugsweise ausgewählt aus Cyclopropyl-, Cyclobutyl-, Cyclopentyl- und Cyclohexylgruppen, besonders bevorzugt ist die C3-C6-Cycloalkylgruppe Cyclopropyl, und/oder die Alkingruppe ein ungesättigter Kohlenwasserstoff mit mindestens einer Kohlenstoff-Kohlenstoff-Dreifachbindung (-C=C-) ist, vorzugsweise ist die Alkingruppe linear, vorzugsweise umfasst die Alkingruppe 2 bis 10 Kohlenstoffatome, vorzugsweise 2 bis 6 Kohlenstoffatome, vorzugsweise 3 Kohlenstoffatome, vorzugsweise ist die Alkingruppe Propinyl.

4. Verbindung nach einem der vorhergehenden Ansprüche, wobei die C1-C20-Alkylgruppe eine lineare oder verzweigte Kohlenwasserstoffgruppe mit 1 bis 20 Kohlenstoffatomen ist, vorzugsweise 2 bis 15 Kohlenstoffatome, oder eine verzweigte Kohlenwasserstoffgruppe mit 3 bis 20 Kohlenstoffatomen, vorzugsweise 3 bis 10 Kohlenstoffatome, und vorzugsweise Ethyl-, n-Propyl-, n-Butyl-, Isobutyl-, n-Pentyl-, n-Hexyl- oder Dodecylgruppen.

5. Verbindung nach einem der vorhergehenden Ansprüche, wobei das Aralkyl eine Gruppe -(CH2)n-Aryl ist, wobei n eine ganze Zahl von 1 bis 5 ist, und Aryl eine monocyclische oder polycyclische aromatische Kohlenwasserstoffgruppe ist, die optional mit mindestens einer Gruppe ausgewählt aus einer Hydroxylgruppe, einer C1-C20-Alkylgruppe und einem Halogen substituiert sein kann, vorzugsweise ist die Aralkylgruppe ein Benzyl, das nicht substituiert oder vorzugsweise in para-Position mit mindestens einer Gruppe ausgewählt aus einer Hydroxylgruppe, einer C1-C6-Alkylgruppe wie Methyl und einem Halogen wie F oder Cl substituiert ist.

6. Verbindung nach einem der vorhergehenden Ansprüche, wobei R eine Hydroxylgruppe oder eine Gruppe -NR¹R² ist, wobei R1 aus einer C1-C20-Alkylgruppe, einer C3-C6-Cycloalkylgruppe, einer Alkingruppe und einer Aralkylgruppe ausgewählt ist, und R² aus H, einer C1-C20-Alkylgruppe und einer Alkingruppe ausgewählt ist; vorzugsweise ist R eine Hydroxylgruppe oder eine Gruppe -NR1R2, wobei R¹ und R² verschieden sind und jeweils unabhängig aus H, einer C3-C6-Cycloalkylgruppe und einer Alkingruppe ausgewählt sind.

7. Verbindung nach Anspruch 1, wobei R eine Hydroxylgruppe ist.

8. Verbindung nach einem der Ansprüche 1 bis 6, wobei R -NR¹R² ist und R² H ist und R¹ eine C3-C6-Cycloalkylgruppe oder eine Alkingruppe ist, vorzugsweise eine C2-C6-Alkingruppe, vorzugsweise ist R² H und R¹ ist Cyclopropyl oder Propinyl.

9. Verbindung nach einem der vorhergehenden Ansprüche, ausgewählt aus folgenden Verbindungen: 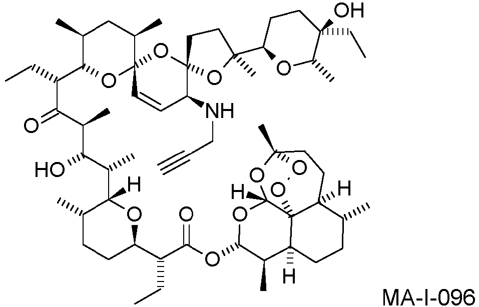 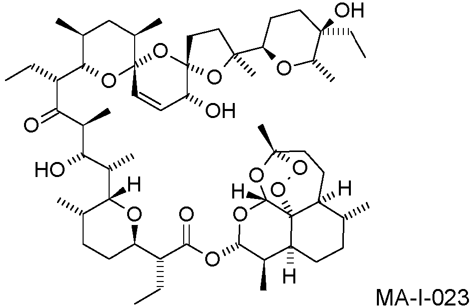 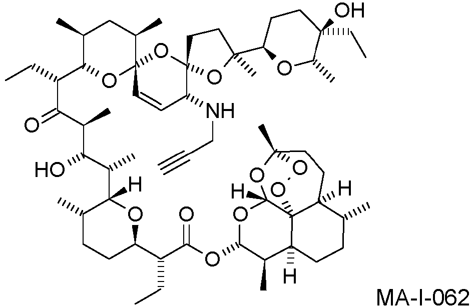 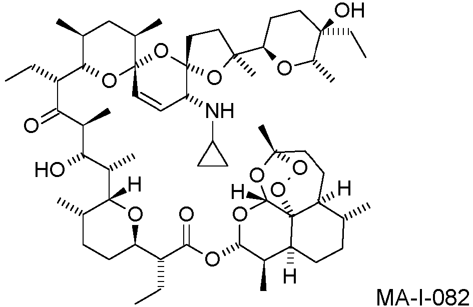 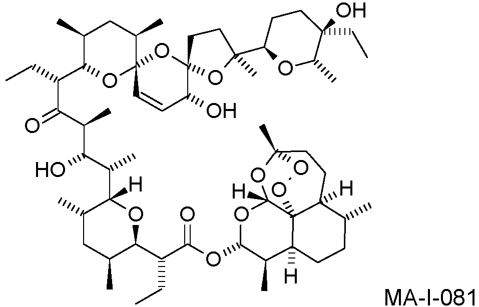 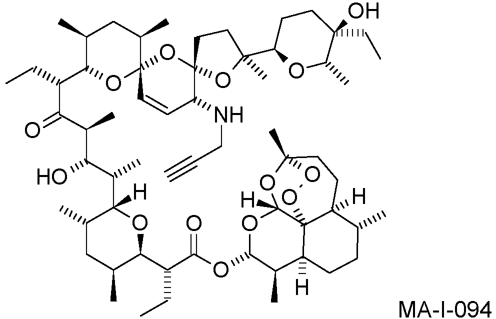 und

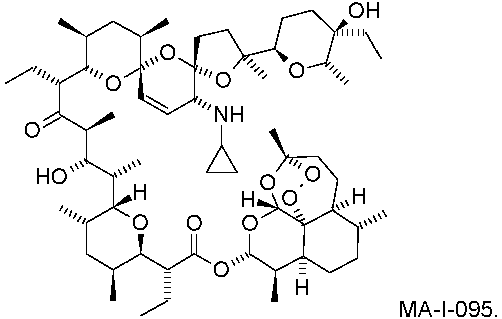

10. Zusammensetzung, umfassend in einem pharmazeutisch verträglichen Medium mindestens eine Verbindung der Formel (I) gemäß einem der vorhergehenden Ansprüche.

11. Verbindung nach einem der Ansprüche 1 bis 9 zur Verwendung als Arzneimittel.

12. Verbindung nach einem der Ansprüche 1 bis 9 zur Verwendung zur Prävention und/oder Behandlung von Krebs und/oder zur Prävention von Metastasen und/oder zur Prävention eines Rückfalls und/oder zur Verringerung der Chemotherapieresistenz bei einem Subjekt, vorzugsweise wobei der Krebs ausgewählt ist aus soliden und nicht-soliden Tumoren, vorzugsweise aus einem Kolonkarzinom, einem kolorektalen Karzinom wie kolorektalen Karzinomen mit einer BRAF-Mutation, insbesondere BRAF V600E, einem Melanom, einem Knochentumor, einem Brustkrebs wie triple-negativem Brustkrebs, einem Schilddrüsenkrebs, einem Prostatakrebs, einem Ovarialkarzinom, einem Lungenkrebs, einem Pankreaskarzinom, einem Gliom wie einem Glioblastom, einem Zervixkarzinom, einem Endometriumkarzinom, einem Kopf-Hals-Tumor, einem Leberkarzinom, einem Blasenkarzinom, einem Nierenkrebs, einem Hautkrebs, einem Magenkarzinom, einem Hodenkrebs, einem Urothelkarzinom oder einem adrenokortikalen Karzinom, Leukämie wie akuter myeloischer Leukämie, Lymphom und multiplem Myelom.

13. Produkt, umfassend: a) eine Verbindung gemäß einem der Ansprüche 1 bis 9, und b) mindestens eine weitere Therapie als Kombinationsprodukt zur gleichzeitigen, getrennten oder sequenziellen Anwendung zur Behandlung von Krebs und/oder zur Prävention von Metastasen und/oder zur Prävention eines Rückfalls und/oder zur Verringerung der Resistenz gegen die weitere Therapie b) bei einem Subjekt.

14. Produkt nach Anspruch 13, wobei die weitere Therapie b) eine Immuntherapie, Chemotherapie und/oder Strahlentherapie ist.

15. Produkt nach Anspruch 13 oder 14, wobei das Subjekt ein Mensch ist, der an Krebs leidet und gegen Chemotherapie resistent ist.

## Revendications

1. Composé choisi parmi les composés de la formule (I) et leurs diastéréo-isomères : 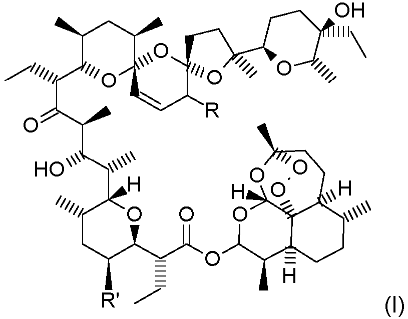
dans laquelle R est un groupe hydroxyle ou un groupe -NR¹R² où R1 et R2 sont identiques ou différents et choisis indépendamment parmi H, un groupe alkyle de C1-C20, un groupe cycloalkyle de C3-C6, un groupe alcyne et un groupe aralkyle, et
dans laquelle R' est H ou méthyle,
de préférence, R1 et R2 sont différents et choisis indépendamment parmi H, un groupe alkyle de C1-C20, un groupe cycloalkyle de C3-C6, un groupe alcyne et un groupe aralkyle.

2. Composé selon la revendication 1, dans lequel il est choisi parmi les composés de la formule (I') et leurs diastéréo-isomères : 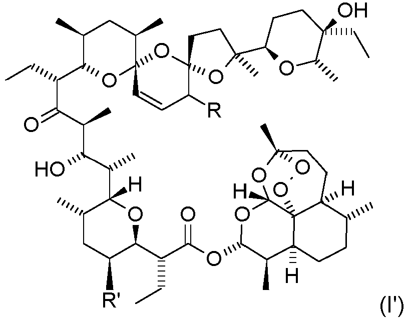 dans laquelle R et R' sont tels que définis ci-dessus.

3. Composé selon l'une quelconque des revendications précédentes, dans lequel le cycloalkyle C3-C6 est un groupe hydrocarboné cyclique comprenant de 3 à 6 atomes de carbone, de préférence il est choisi parmi les groupes cyclopropyle, cyclobutyle, cyclopentyle, et cyclohexyle, plus préférentiellement le cycloalkyle C3-C6 est le cyclopropyle, et/ou le groupe alcyne est un hydrocarbure insaturé comprenant au moins une triple liaison carbone-carbone (-C=C-), de préférence le groupe alcyne est linéaire, de préférence le groupe alcyne comprend de 2 à 10 atomes de carbone, de préférence de 2 à 6 atomes de carbone, de préférence 3 atomes de carbone, de préférence le groupe alcyne est le propynyle.

4. Composé selon l'une quelconque des revendications précédentes, dans lequel le groupe alkyle C1-C20 est un groupe linéaire ou hydrocarboné comprenant de 1 à 20 atomes de carbone, de préférence de 2 à 15 atomes de carbone, ou un groupe hydrocarboné ramifié comprenant de 3 à 20 atomes de carbone, de préférence de 3 à 10 atomes de carbone, et de préférence des groupes éthyle, n-propyle, n-butyle, isobutyle, n-pentyle, n-hexyle ou dodécyle.

5. Composé selon l'une quelconque des revendications précédentes, dans lequel l'aralkyle est un groupe -(CH₂) n-aryle, dans lequel n est un nombre entier de 1 à 5, et l'aryle est un groupe hydrocarboné aromatique monocyclique ou polycyclique, qui peut être éventuellement substitué par au moins un groupe choisi parmi un groupe hydroxyle, un groupe alkyle en C1-C20 et un halogène, de préférence le groupe aralkyle est un benzyle non substitué ou substitué, de préférence en position para, par au moins un groupe choisi parmi un groupe hydroxyle, un groupe alkyle en C1-C6 tel que le méthyle, et un halogène tel que F ou Cl.

6. Composé selon l'une quelconque des revendications précédentes, dans lequel R est un groupe hydroxyle ou un groupe -NR1R2, où R1 est choisi parmi un groupe alkyle en C1-C20, un groupe cycloalkyle en C3-C6, un groupe alcyne et un groupe aralkyle, et R2 est choisi parmi H, un groupe alkyle en C1-C20 et un groupe alcyne ; de préférence, R est un groupe hydroxyle ou un groupe -NR1R2 où R1 et R2 sont différents et choisis indépendamment parmi H, un groupe cycloalkyle en C3-C6 et un groupe alcyne.

7. Composé selon la revendication 1, dans lequel R est un groupe hydroxyle.

8. Composé selon l'une des revendications 1 à 6, dans lequel R est -NR1R2, et R2 est H et R1 est un groupe cycloalkyle C3-C6 ou un groupe alcyne, de préférence un groupe alcyne C2-C6, de préférence R2 est H et R1 est cyclopropyle ou propynyle.

9. Composé selon l'une quelconque des revendications précédentes, dans lequel il est choisi parmi les composés suivants : 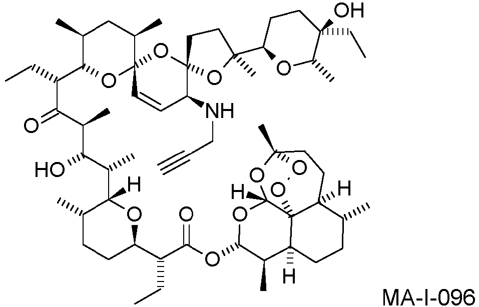 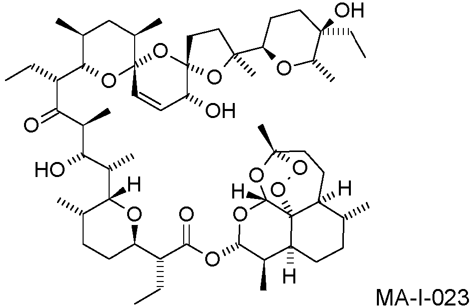 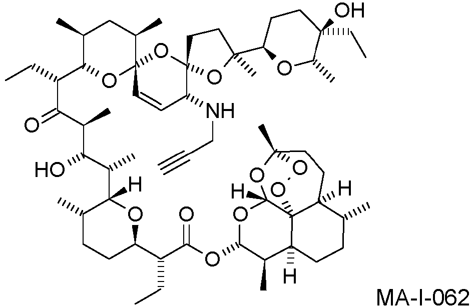 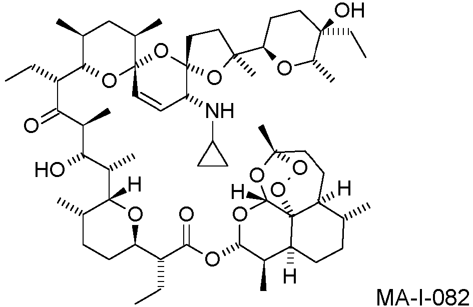 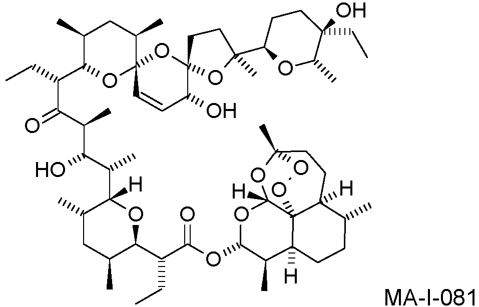 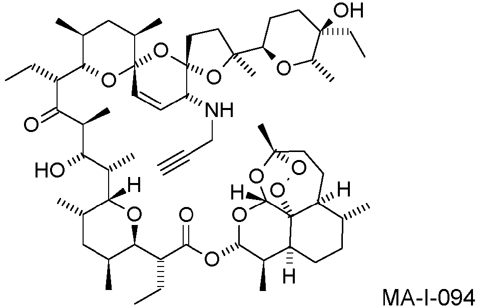 et

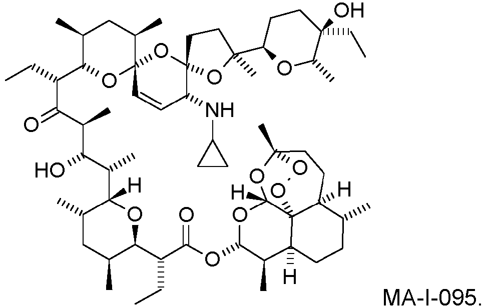

10. Composition comprenant, dans un milieu pharmaceutiquement acceptable, au moins un composé de formule (1) selon l'une quelconque des revendications précédentes.

11. Composé selon l'une des revendications 1 à 9, destiné à être utilisé comme médicament.

12. Composé selon l'une quelconque des revendications 1 à 9, pour utilisation pour la prévention et/ou le traitement du cancer, et/ou pour la prévention des métastases du cancer et/ou pour la prévention de la récurrence du cancer et/ou pour la diminution de la résistance à une chimiothérapie chez un sujet , de préférence dans lequel le cancer est choisi parmi les cancers solides et non solides, de préférence parmi un cancer du côlon, un cancer colorectal tel que les cancers colorectaux avec une mutation BRAF en particulier BRAF V600E, un mélanome, un cancer de l'os, un cancer du sein tel que le cancer du sein triple négatif, un cancer de la thyroïde, un cancer de la prostate, un cancer des ovaires, un cancer du poumon, un cancer du pancréas, un gliome tel qu'un glioblastome, un cancer du col de l'utérus, un cancer de l'endomètre, un cancer de la tête et du cou, un cancer du foie, un cancer de la vessie, un cancer du rein, un cancer de la peau, un cancer de l'estomac, un cancer du testicule, un cancer urothélial ou un carcinome corticosurrénalien, une leucémie telle que la leucémie myéloïde aiguë, un lymphome et un myélome multiple.

13. Produit comprenant :
a) un composé selon l'une des revendications 1 à 9, et
b) au moins une thérapie supplémentaire,
en tant que produit combiné pour une utilisation simultanée, séparée ou séquentielle pour traiter le cancer, et/ou pour prévenir les métastases du cancer, et/ou pour prévenir la récurrence du cancer, et/ou pour diminuer la résistance à la thérapie supplémentaire b), chez un sujet.

14. Produit selon la revendication 13, dans lequel la thérapie supplémentaire b) est une immunothérapie, une chimiothérapie et/ou une radiothérapie.

15. Le produit selon la revendication 13 ou 14, dans lequel le sujet est un humain souffrant d'un cancer et résistant à la chimiothérapie.
